(19) Europäisches Patentamt
European Patent Office
Office européen des brevets

(11) **EP 2 843 393 B1**

(12) **EUROPEAN PATENT SPECIFICATION**

(45) Date of publication and mention
of the grant of the patent:
**31.01.2018 Bulletin 2018/05**

(21) Application number: **13781395.2**

(22) Date of filing: **26.04.2013**

(51) Int Cl.:
**G01N 21/53** *(2006.01)*          **G01N 21/85** *(2006.01)*
**G01N 33/28** *(2006.01)*          **B25J 19/00** *(2006.01)*
**B25J 19/02** *(2006.01)*

(86) International application number:
**PCT/JP2013/062500**

(87) International publication number:
**WO 2013/162031 (31.10.2013 Gazette 2013/44)**

(54) **LUBRICATING OIL DEGRADATION SENSOR AND MACHINE PROVIDED THEREWITH**

SCHMIERÖLVERSCHLEISSSENSOR UND DAMIT AUSGERÜSTETE MASCHINE

CAPTEUR DE DÉGRADATION D'HUILE LUBRIFIANTE ET MACHINE L'UTILISANT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **26.04.2012 JP 2012101900**

(43) Date of publication of application:
**04.03.2015 Bulletin 2015/10**

(73) Proprietor: **Nabtesco Corporation
Tokyo 102-0093 (JP)**

(72) Inventors:
• **NAKAMURA, Koji
Tsu-shi, Mie 514-8533 (JP)**
• **SHIMADA, Hideshi
Tsu-shi, Mie 514-8533 (JP)**

(74) Representative: **Grünecker Patent- und
Rechtsanwälte
PartG mbB
Leopoldstraße 4
80802 München (DE)**

(56) References cited:
**WO-A1-2012/000539        JP-A- H0 886 751
JP-A- H02 111 232        JP-A- H04 242 153
JP-A- H07 146 233        JP-A- H09 138 196
JP-A- H11 235 097        JP-A- 2005 156 297
JP-A- 2008 073 775        JP-U- H0 534 562
US-A1- 2008 024 761        US-A1- 2010 045 989
US-B1- 6 559 655**

EP 2 843 393 B1

**Description**

Technical Field

The present invention relates to a lubricant deterioration sensor for detecting the deterioration of lubricant of a machine.

Background Art

Conventionally, as a lubricant deterioration sensor for detecting the deterioration of lubricant of a machine, an oil deterioration degree sensor has been known. In this sensor, an oil entering the gap portion for entering lubricant therein is formed on an optical path from an infrared LED (Light Emitting Diode) to a photo diode. Then, an amount of light absorption by the lubricant within the oil entering the gap portion with respect to light emitted from the infrared LED is measured according to a light reception amount of the photo diode, to thereby determine a deterioration degree of the lubricant related to the light absorption amount thus measured (see PTLs 1 and 2, for example).

However, the oil deterioration degree sensor described in each of PTLs 1 and 2 has a problem that, although a density of insoluble content within the lubricant can be measured as the deterioration degree of the lubricant, kinds of contaminant within the lubricant cannot be specified.

As a technique of specifying the kinds of contaminant within lubricant, a technique has been known in which an LED irradiates light toward a membrane filter having been used to filter the lubricant. Then, a light reception element converts reflection light from the contaminant on the membrane filter into digital values of RGB, to thereby specify the kinds of contaminant within the lubricant based on the RGB digital values thus converted (see NPLs 1 and 2, for example).

Citation List

Patent Literature

[PTL 1] JP-A-7-146233
[PTL 2] JP-A-10-104160
[PLT 3] US 2010/0045989 A1
[PLT 4] US 2008/0024761 A1

PLT 3 discloses a device for monitoring the condition of a medium, comprising a measuring head adapted to be pushed into the wall of a channel comprising lubricating or hydraulic oils.

PLT 4 discloses an apparatus for monitoring oil deterioration in real time.

Non Patent Literature

[NPL 1] Tomohiko Yamaguchi and four others, "METHOD OF DISCRIMINATING HUE OF CONTAMINANT WITHIN LUBRICANT", Fukui University, Faculty of Engineering, Research Report, March 2003, Volume 51, No. 1, pp. 81-88
[NPL 2] Tomomi Honda, "DETERIORATION DIAGNOSIS OF LUBRICANT·INSPECTION TECHNOLOGY", Journal of Japan Society for Precision Engineering, 2009, Volume 75, No. 3, pp. 359 - 362

Summary of Invention

Technical Problem

Since the technique described in each of the [NPL 1] and [NPL 2] requires to drain lubricant from a machine and filter the lubricant by a membrane filter, there arises a problem of lacking immediacy.

An object of this invention is to provide a lubricant deterioration sensor which enables to immediately specify the kinds and amounts of contaminant within lubricant of a machine.

Solution to Problem

According to advantages of the invention, there is provided a lubricant deterioration sensor which is installed in a machine body and adapted to detect deterioration of lubricant of the machine body, the sensor including: a light emitting element which is configured to emit light; a color light reception element which is configured to detect a color of received light; a circuit board on which the light emitting element and the color light reception element are mounted; a gap forming member at which an oil gap for allowing the lubricant to enter therein is formed; a supporting member which supports the light emitting element, the color light reception element, the circuit board, and the gap forming member; a fixing member which is configured to be fixed to the machine body; and a shock absorbing member which is disposed between the supporting member and the fixing member and is configured to absorb shocks, wherein the gap forming member is configured to transmit the light emitted from the light emitting element, the oil gap is disposed on an optical path from the light emitting element to the color light reception element, the supporting member is provided with a substrate attaching portion having the circuit board attached thereon, and at least a part of the shock absorbing member is disposed in a peripheral space of the substrate attaching portion which is formed by surrounding the substrate attaching portion between the supporting member and the fixing member.

The shock absorbing member may be provided with a fixing member adhesion portion which is a continuous portion surrounding the supporting member and adheres to the fixing member and a supporting member adhesion portion which is an integrated portion formed by surrounding the supporting member and adheres to the supporting member.

The fixing member may include a contact portion that comes in contact with the machine body on an outer side thereof, the supporting member may be disposed on an inner side of the contact portion in a manner in which at least a part of the optical path is disposed on the inner side of the contact portion, and the shock absorbing member may be disposed in a manner in which at least a part thereof is disposed between the contact portion and the supporting member.

The contact portion may be a portion configured to be inserted into a hole of the machine body.

The hole may be a screw hole, and the contact portion may be a screw portion which is configured to be inserted into the screw hole of the machine body and fixed thereto.

According to this invention, the machine may be provided with the lubricant deterioration sensor and the machine body.

Further, according to this invention, the machine may be a reducer for an industrial robot, and the machine body may be a main body of the reducer.

Still further, according to this invention, the machine may be the industrial robot, and provided with an arm and a reducer used at the articular portion of the arm, and the lubricant may be lubricant for the reducer.

Advantageous Effects of Invention

In the lubricant deterioration sensor according to this invention, the color light reception element detects colors with respect to the light having wavelengths not absorbed by the contaminant such as ion powder within the lubricant at the oil gap, among the light emitted by the light emitting element. Thus, colors of the contaminant within the lubricant of the machine body can be detected immediately. That is, the lubricant deterioration sensor according to this invention enables to immediately specify the kinds and amounts of the contaminant within the lubricant of the machine body based on the colors detected by the color light reception element. In addition, the lubricant deterioration sensor according to this invention enables absorption of the shocks transferred to the substrate attaching portion of the support member from the fixing member by the shock absorbing member which is disposed between the supporting member and the fixing member when the fixing member is subjected to shocks from the machine body, and thus it is possible to suppress a possibility of damaging an electronic component mounted on the circuit board which is attached to the substrate attaching portion due to the shocks. Therefore, the lubricant deterioration sensor according to this invention enables suppression of a failure occurring when the fixing member is subjected to shocks from the machine body. In addition, the lubricant deterioration sensor according to this invention enables absorption of the shocks transferred to the substrate attaching portion of the support member from the fixing member by the shock absorbing member which is disposed between the supporting member and the fixing member when the fixing member is subjected to shocks from the machine body, and thus it is possible to suppress a possibility of changing a positional relationship of the light emitting element and the color light reception element which are mounted on the circuit board attached on the substrate attaching portion due to the shocks. Accordingly, in the case where the contact portion is made in contact with the machine body, the detection accuracy of the deterioration of the lubricant of the machine body can be suppressed from degrading.

The lubricant deterioration sensor according to this invention not only enables absorption of the shocks transferred to the support member from the fixing member by the shock absorbing member but also prevention of the leakage of lubricant from between the fixing member and the supporting member by the shock absorbing member.

Further, in the lubricant deterioration sensor according to this invention, even if the contact portion is made in contact with the machine body and is deformed, the deformation of the contact portion is hardly transmitted to the supporting member by the shock absorbing member which is disposed between the contact portion and the supporting member. Thus, the change of the optical path due to the deformation of the supporting member hardly occurs. Accordingly, in the case where the contact portion is made in contact with the machine body, the detection accuracy of the deterioration of the lubricant of the machine body can be suppressed from degrading.

In the lubricant deterioration sensor according to this invention, the contact portion likely deforms toward the inside thereof when the contact portion is inserted into the hole of the machine body and made in contact with the machine body. Thus, in the case where the contact portion is made in contact with the machine body, the detection accuracy of the deterioration of the lubricant of the machine body can be efficiently suppressed from degrading.

In the lubricant deterioration sensor according to this invention, the screw portion likely deforms toward the inside thereof when the screw portion is threaded into the screw hole of the machine body and made in contact with the machine body. Thus, in the case where the screw portion is fixed to the machine body, the detection accuracy of the deterioration of the lubricant of the machine body can be efficiently suppressed from degrading.

The machine according to this invention can immediately specify the kinds and amounts of the contaminant within the lubricant of the machine body by using the lubricant deterioration sensor. Further, the machine according to this invention can suppress a possibility of the failure of the lubricant deterioration sensor in a case where the lubricant deterioration sensor is subjected to shocks from the machine body and a possibility of the degradation of the detection accuracy of deterioration of the lubricant of the machine body by the lubricant deterioration sensor.

The reducer for the industrial robot according to this invention can suppress the possibility of failure or the degradation of the detection of the lubricant deterioration sensor which enables to immediately specify the kinds and amounts of the contaminant within the lubricant. Thus, the reducer for the industrial robot according to this invention can maintain for a long time the accuracy of the immediate prediction as to a failure. In addition, the reducer for the industrial robot according to this invention, when being attached to the industrial robot, receives the acceleration in accordance with an operation of the industrial robot or receives a vibration by the vibration of the industrial robot, and thus is subjected to shocks generated due to vibration and acceleration for a long time. Accordingly, the reducer for the industrial robot according to this invention can suppress a possibility of the failure of the lubricant deterioration sensor in a case where the lubricant deterioration sensor is subjected to shocks from the machine body and a possibility of the degradation of the detection accuracy of deterioration of the lubricant of the machine body by the lubricant deterioration sensor.

The industrial robot according to this invention can suppress the possibility of failure or the degradation of the detection of the lubricant deterioration sensor which enables to immediately specify the kinds and amounts of the contaminant within the lubricant. Thus, the reducer for the industrial robot according to this invention can maintain for a long time the accuracy of the immediate prediction as to a failure. In addition, the industrial robot according to this invention gives the lubricant deterioration sensor the acceleration in accordance with the operation of the industrial robot itself or gives the lubricant deterioration sensor a vibration by the vibration of the industrial robot, and thus gives the lubricant deterioration sensor shocks generated due to vibration and acceleration for a long time. Accordingly, the industrial robot according to this invention can suppress a possibility of the failure of the lubricant deterioration sensor in a case where the lubricant deterioration sensor is subjected to shocks from the machine body and a possibility of the degradation of the detection accuracy of deterioration of the lubricant of the machine body by the lubricant deterioration sensor.

Hereinafter, the lubricant deterioration sensor according to this invention enables to immediately specify the kinds and amounts of the contaminant within the lubricant of the machine.

Brief Description of Drawings

Fig. 1 is a side view of an industrial robot according to the first embodiment of the present invention.
Fig. 2 is a sectional view of the articular portion of the industrial robot shown in Fig. 1.
Fig. 3 is a front view of a lubricant deterioration sensor shown in Fig. 2.
Fig. 4 is a front sectional view of the lubricant deterioration sensor shown in Fig. 3 in an attached state to an arm.
Fig. 5(a) is a plan view of the lubricant deterioration sensor shown in Fig. 3. Fig. 5(b) is a bottom view of the lubricant deterioration sensor shown in Fig. 3.
Fig. 6(a) is a front view of a housing shown in Fig. 3. Fig. 6(b) is a front sectional view of the housing shown in Fig. 3.
Fig. 7(a) is a side view of the housing shown in Fig. 3. Fig. 7(b) is a side sectional view of the housing shown in Fig. 3.
Fig. 8(a) is a plan view of the housing shown in Fig. 3. Fig. 8(b) is a bottom view of the housing shown in Fig. 3.
Fig. 9(a) is a front view of a holder shown in Fig. 4. Fig. 9(b) is a front sectional view of the holder shown in Fig. 4.
Fig. 10(a) is a side view of the holder shown in Fig. 4. Fig. 10(b) is a side sectional view of the holder shown in Fig. 4.
Fig. 11 (a) is a plan view of the holder shown in Fig. 4. Fig. 11 (b) is a bottom view of the holder shown in Fig. 4.
Fig. 12 is a diagram showing an optical path from a white LED to an RGB sensor shown in Fig. 4.

Fig. 13(a) is a front view of a holder cap shown in Fig. 4. Fig. 13(b) is a front sectional view of the holder cap shown in Fig. 4.

Fig. 14(a) is a plan view of the holder cap shown in Fig. 4. Fig. 14(b) is a bottom view of the holder cap shown in Fig. 4.

Fig. 15(a) is a front view of a shock absorbing member shown in Fig. 4. Fig. 15(b) is a front sectional view of the shock absorbing member in Fig. 4.

Fig. 16 is a diagram showing an example of the relation between the direction of the opening of an oil gap shown in Fig. 3 with respect to the flow of lubricant and a color difference ∆E of colors detected by the RGB sensor with respect to black.

Fig. 17(a) is a diagram showing a state that the direction of the opening of the oil gap shown in Fig. 3 with respect to the flow of the lubricant is 0 degree. Fig. 17(b) is a diagram showing a state that the direction of the opening of the oil gap shown in Fig. 3 with respect to the flow of the lubricant is 45 degrees. Fig. 17(c) is a diagram showing a state that the direction of the opening of the oil gap shown in Fig. 3 with respect to the flow of the lubricant is 90 degrees.

Fig. 18 is a front sectional view of the lubricant deterioration sensor of the industrial robot according to a second embodiment of the invention in a state where the lubricant deterioration sensor is attached to the arm.

Fig. 19(a) is a plan view of the lubricant deterioration sensor shown in Fig. 18. Fig. 19(b) is a bottom view of the lubricant deterioration sensor shown in Fig. 18.

Fig. 20 is a cross-sectional view taken along line I-I of Fig. 18.

Fig. 21 (a) is a front sectional view of a housing shown in Fig. 18. Fig. 21(b) is a side cross-sectional view of the housing shown in Fig. 18.

Fig. 22(a) is a plan view of the housing shown in Fig. 18. Fig. 22(b) is a bottom view of the housing shown in Fig. 18.

Fig. 23(a) is a front view of a holder shown in Fig. 18. Fig. 23(b) is a side view of the holder shown in Fig. 18.

Fig. 24 is a bottom view of the holder shown in Fig. 18.

Fig. 25 is a front sectional view of the lubricant deterioration sensor of the industrial robot according to a third embodiment of the invention in a state where the lubricant deterioration sensor is attached to the arm.

Fig. 26(a) is a front sectional view of the housing shown in Fig. 25. Fig. 26(b) is a plan view of the housing shown in Fig. 25.

Fig. 27(a) is a front view of a holder shown in Fig. 25. Fig. 27(b) is a front sectional view of the holder shown in Fig. 25.

Fig. 28 is a bottom view of the holder shown in Fig. 25.

Fig. 29 is a front sectional view of the lubricant deterioration sensor of the industrial robot according to a fourth embodiment of the invention in a state where the lubricant deterioration sensor is attached to the arm.

Fig. 30(a) is a front sectional view of the housing shown in Fig. 29. Fig. 30(b) is a plan view of the housing shown in Fig. 29.

Fig. 31 (a) is a front view of a holder shown in Fig. 29. Fig. 31 (b) is a front sectional view of the holder shown in Fig. 29.

Fig. 32 is a bottom view of the holder shown in Fig. 29.

Description of Embodiments

Hereinafter, embodiments of this invention will be explained with reference to drawings.

First, the configuration of an industrial robot as a machine according to the first embodiment will be explained.

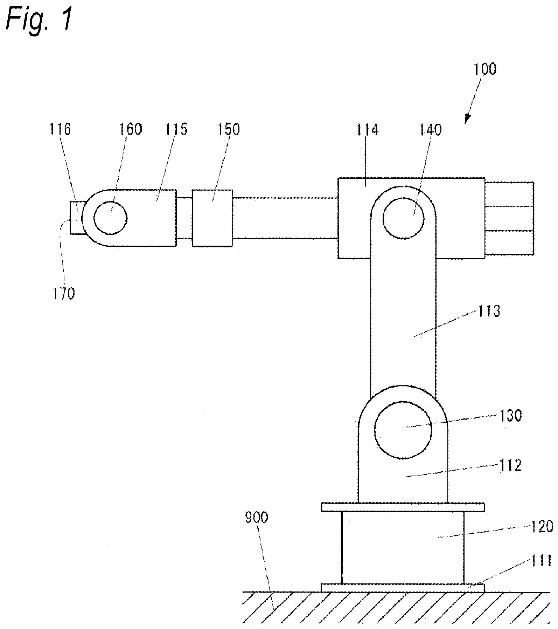

Fig. 1 is a side view of the industrial robot 100 according to this embodiment.

As shown in Fig. 1, the industrial robot 100 includes an attachment portion 111 to be attached to an installation portion 900 such as a floor or a ceiling, arms 112 to 116, an articular portion 120 for connecting between the attachment portion 111 and an arm 112, an articular portion 130 for connecting between the arm 112 and an arm 113, an articular portion 140 for connecting between the arm 113 and the arm 114, an articular portion 150 for connecting between the arm 114 and the arm 115, an articular portion 160 for connecting between the arm 115 and the arm 116, and an articular portion 170 for connecting between the arm 116 and a not-shown hand.

Of the industrial robot 100, portions except for lubricant such as lubricant 131a described later, the lubricant deterioration sensors such as lubricant deterioration sensors 137a, 137b, 139a, 139b described later constitute a machine body according to this invention in a case where the industrial robot 100 is the machine according to this invention.

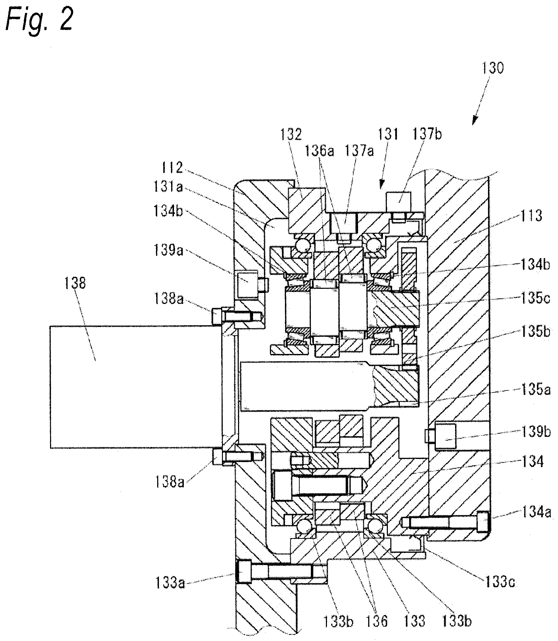

Fig. 2 is a sectional view of the articular portion 130. Although the explanation is made hereinafter as to the articular portion 130, the configuration of each of the articular portions 120, 140 to 170 is substantially same.

As shown in Fig. 2, the articular portion 130 includes a reducer 131, which is the reducer for the industrial robot, for connecting between the arm 112 and the arm 113, a motor 138 fixed to the arm 112 by means of bolts 138a, and the lubricant deterioration sensors 139a, 139b each for detecting the deterioration of the lubricant 131a for reducing friction generated at the movable portions of the reducer 131.

The reducer 131 includes a reducer body 132 and the lubricant deterioration sensors 137a and 137b each for detecting the deterioration of the lubricant 131a of the reducer body 132. The reducer body 132 constitutes a machine body according to this invention in a case where the reducer 131 is the machine according to this invention.

The reducer body 132 includes a case 133 fixed to the arm 112 by means of bolts 133a; a supporting body 134 fixed to the arm 113 by means of bolts 134a; a gear 135a fixed to the output shaft of the motor 138; three gears 135b which are disposed around the center axis of the reducer 131 with a constant interval therebetween and mesh with the gear 135a; three crank shafts 135c which are disposed around the center axis of the reducer 131 with a constant interval therebetween and fixed to the gears 135b, respectively; and two external gears 136 which mesh with internal gears provided at the case 133.

The supporting body 134 is rotatably supported by the case 133 via bearings 133b. A seal member 133c for preventing leakage of the lubricant 131a is provided between the case 133 and the supporting body 134.

Each of the crank shafts 135c is rotatably supported by the supporting body 134 via bearings 134b and further rotatably supported by the external gears 136 via bearings 136a.

Each of the lubricant deterioration sensor 137a and the lubricant deterioration sensor 137b is fixed to the case 133. The lubricant deterioration sensor 139a is fixed to the arm 112. The lubricant deterioration sensor 139b is fixed to the arm 113.

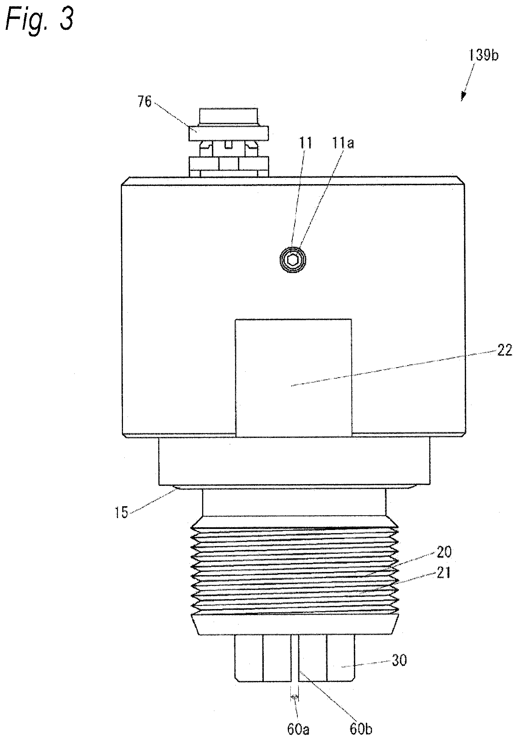

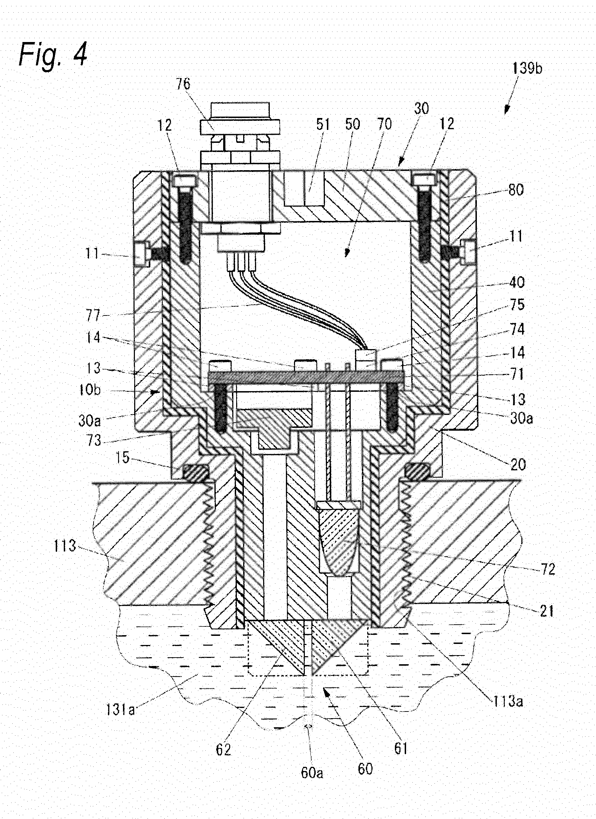

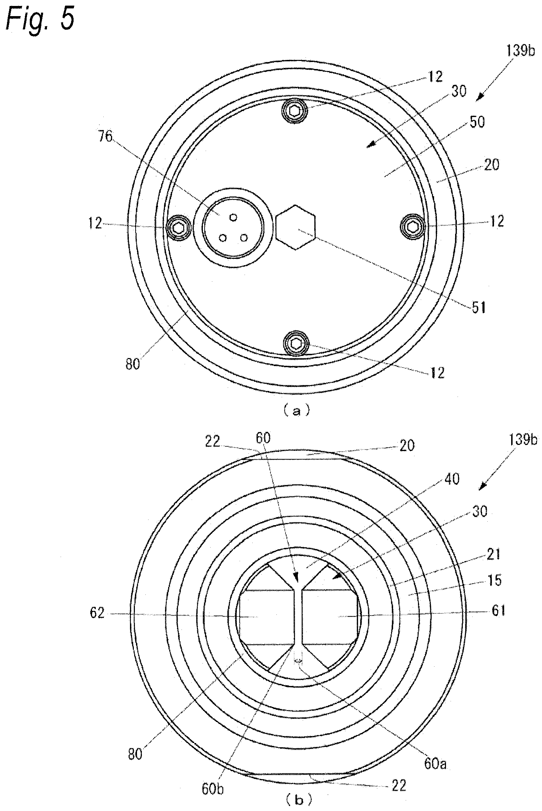

Fig. 3 is a front view of the lubricant deterioration sensor 139b. Fig. 4 is a front sectional view of the lubricant deterioration sensor 139b in an attached state to the arm 113. Fig. 5(a) is a plan view of the lubricant deterioration sensor 139b. Fig. 5(b) is a bottom view of the lubricant deterioration sensor 139b. Although the explanation is made hereinafter as to the lubricant deterioration sensor 139b, the configuration of each of the lubricant deterioration sensors such as the lubricant deterioration sensors 137a, 137b, and 139a other than the lubricant deterioration sensor 139b is substantially same.

As shown in Figs. 3 to 5(b), the lubricant deterioration sensor 139b includes a housing 20 made of an aluminum alloy for supporting the respective components of the lubricant deterioration sensor 139b; a supporting member 30 for supporting a gap forming member 60 described later; the gap forming member 60 held by the supporting member 30; an electronic component group 70; and a shock absorbing member 80 which is disposed between the housing 20 and the supporting member 30 and made of soft rubber which absorbs shocks.

The supporting member 30 is fixed to the housing 20 by means of bolts 11 with hexagon holes. The supporting member 30 includes a holder 40 made of an aluminum alloy, and a holder cap 50 made of an aluminum alloy which is fixed to the holder 40 by means of bolts 12 with hexagon holes.

The gap forming member 60 is constituted by two right-angle prisms 61, 62 made of glass. An oil gap 60a as a gap for entering the lubricant 131a therein is formed between the two right-angle prisms 61, 62.

The electronic component group 70 includes a circuit board 71 fixed to the supporting member 30 by means of bolts 14 with hexagon holes via a spacer 13; a white LED 72 mounted on the circuit board 71; an RGB sensor 73 mounted on the circuit board 71; a connector 74 mounted on the circuit board 71 opposing the white LED 72 and the RGB sensor 73 side; a connector 75 capable of electrically connecting to the connector 74; a water proof connector 76 fixed to the holder cap 50; and a plurality of lead lines 77 which electrically connect the connector 75 and the water proof connector 76 to each other. In addition to the white LED 72, the RGB sensor 73 and the connector 74, a plurality of electronic components are mounted such as an electronic component which processes the signals of the white LED 72 and the RGB sensor 73 on the circuit board 71. The water proof connector 76 is connected to the connector of the external device of the lubricant deterioration sensor 139b so as to be supplied with electric power from the external device via the connector of the external device, and the detection result of the lubricant deterioration sensor 139b is output as an electric signal to the external device via the connector of the external device.

The lubricant deterioration sensor 139b includes an O ring 15 for preventing the leakage of the lubricant 131a from a gap between the housing 20 and the arm 113.

A bolt 11 with hexagon holes is configured so as to prevent the rotation of the supporting member 30 with respect to the housing 20 by coming in contact with both the supporting member 30 and the housing 20. The bolt 11 with hexagon holes includes a tool contact portion 11a for connecting the tool such as a hexagonal wrench. The tool contact portion 11a acts as the portion for receiving the driving force for coming in contact with both the supporting member 30 and the housing 20 from the outside by the contact force. The tool contact portion 11a is disposed at the position not in contact with the lubricant 131a when the housing 20 is fixed to the arm 113.

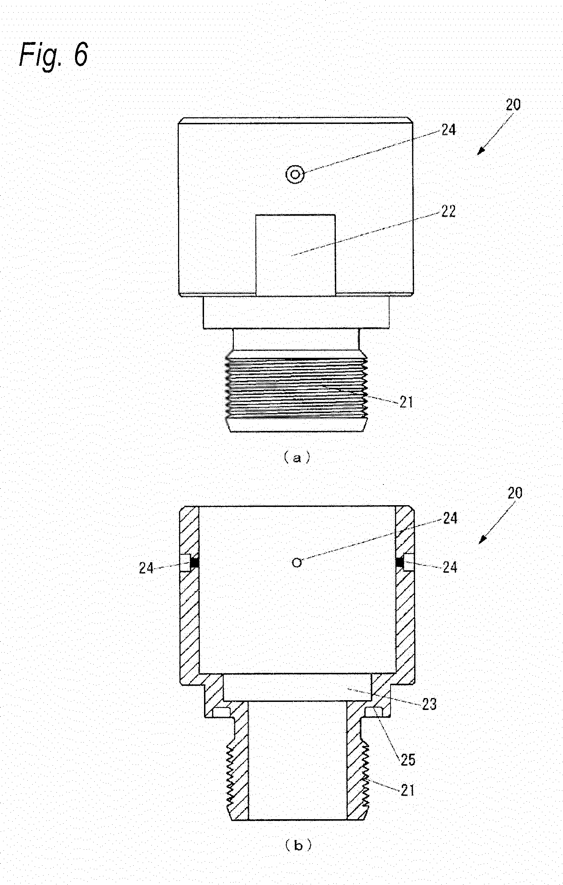

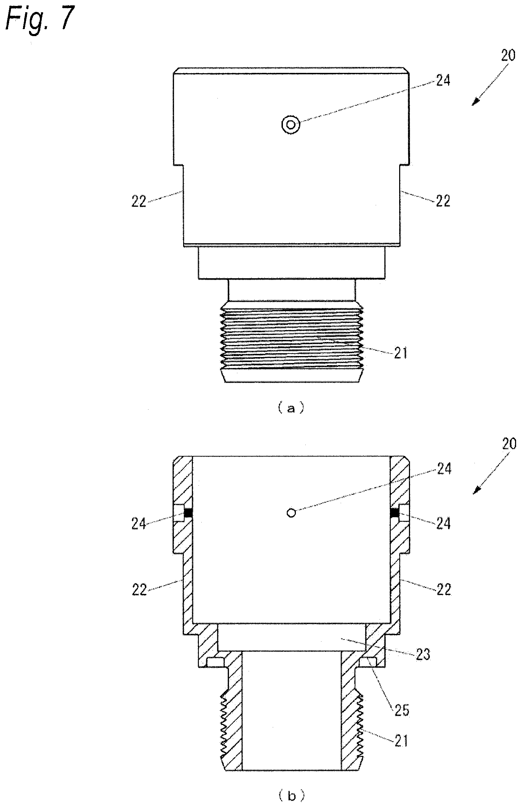

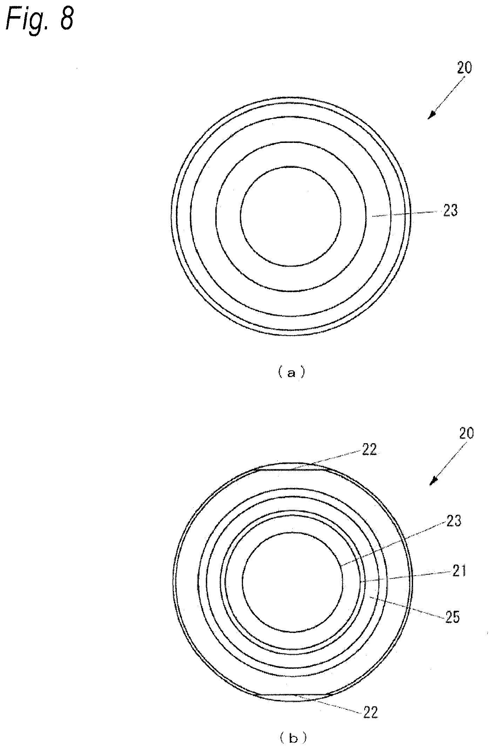

Fig. 6(a) is a front view of the housing 20. Fig. 6(b) is a front sectional view of the housing 20. Fig. 7(a) is a side view of the housing 20. Fig. 7(b) is a side sectional view of the housing 20. Fig. 8(a) is a plan view of the housing 20. Fig. 8(b) is a bottom view of the housing 20.

As shown in Figs. 3 to 8(b), the housing 20 includes a screw portion 21 to be fixed to the screw hole 113a of the arm 113, a tool contact portion 22 which is grasped by a tool such as a wrench at the time of rotating the screw portion 21 with respect to the screw hole 113a of the arm 113, and a holder housing portion 23 in which the holder 40 is housed. Further, the housing 20 is provided with screw holes 24 into which the bolts 11 with hexagon holes are respectively screwed, and a groove 25 into which the O ring 15 is fit.

The housing 20 is configured to be fixed to the arm 113 of the industrial robot 100, that is, the machine body and hence constitutes a fixing member of this invention. Further, the screw portion 21 is configured to be inserted into

the screw hole 113a of the arm 113 and fixed to the screw hole 113a on the outer side thereof, and constitutes a contact portion according to this invention.

The screw hole 113a of the arm 113 may be used for supplying the lubricant 131a to the reducer 131 and for disposing the lubricant 131a from the reducer 131 in a state that the lubricant deterioration sensor 139b is removed.

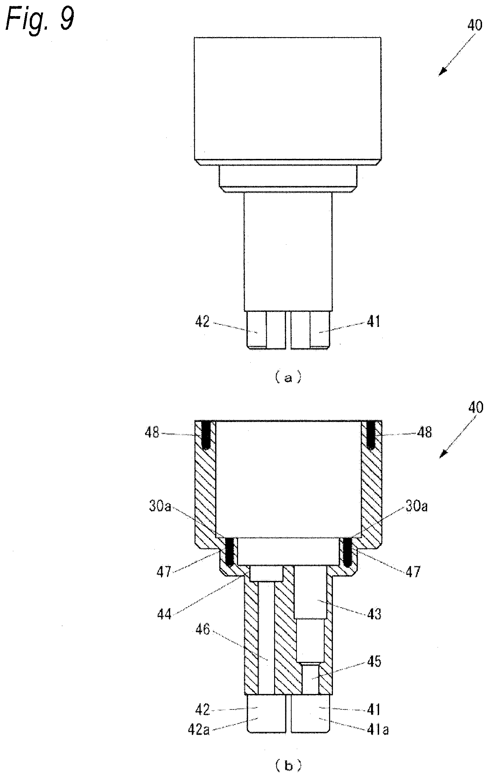

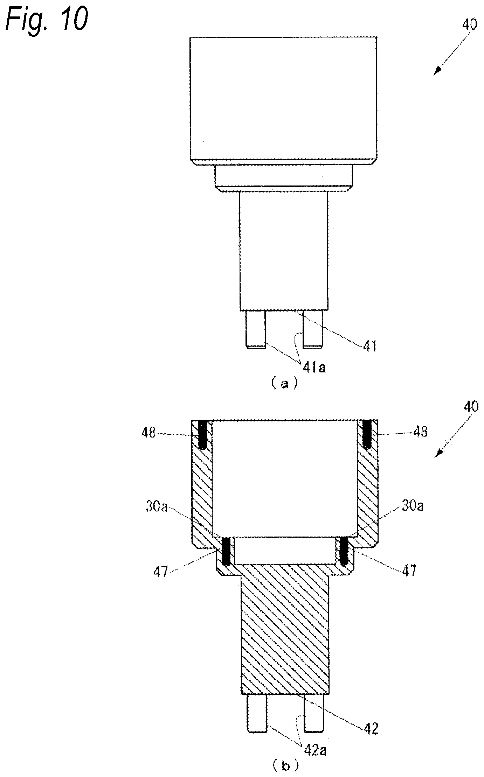

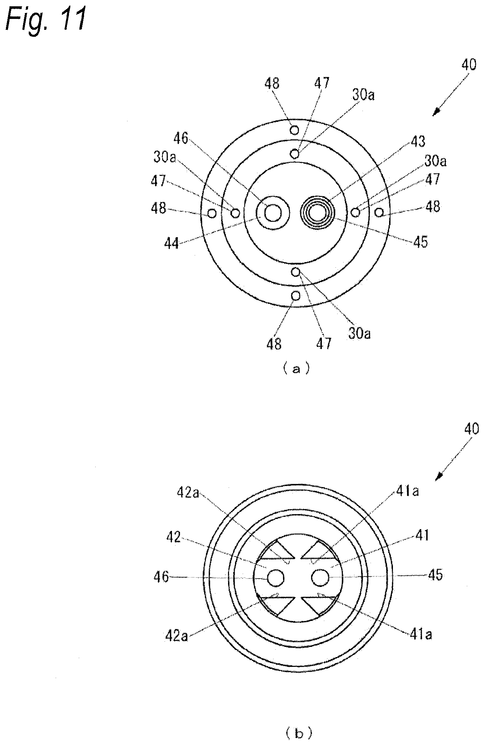

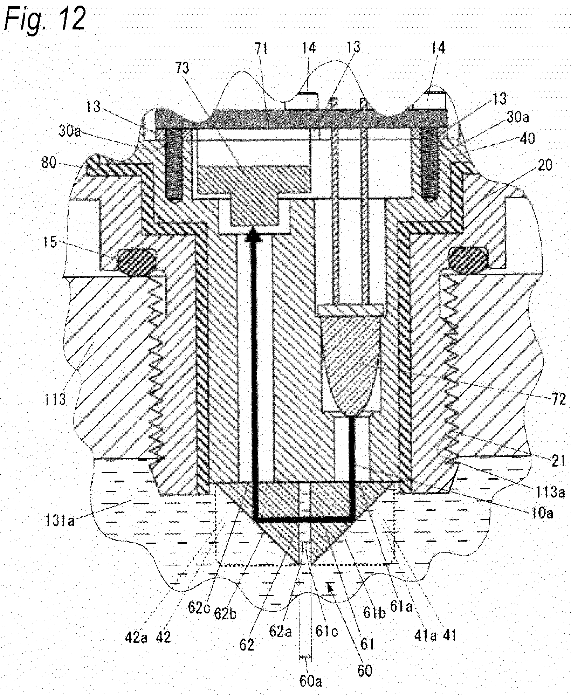

Fig. 9(a) is a front view of the holder 40. Fig. 9(b) is a front sectional view of the holder 40. Fig. 10(a) is a side view of the holder 40. Fig. 10(b) is a side sectional view of the holder 40. Fig. 11 (a) is a plan view of the holder 40. Fig. 11 (b) is a bottom view of the holder 40. Fig. 12 is a diagram showing an optical path 10a from the white LED 72 to the RGB sensor 73.

As shown in Figs.3 to 5(b) and Figs. 9(a) to 12, the holder 40 includes a prism housing portion 41 for housing the right-angle prism 61, a prism housing portion 42 for housing the right-angle prism 62, an LED housing portion 43 for housing the white LED 72, and an RGB sensor housing portion 44 for housing the RGB sensor 73. Further, the holder 40 is provided with a hole 45 penetrating the prism housing portion 41 and the LED housing portion 43, a hole 46 penetrating the prism housing portion 42 and the RGB sensor housing portion 44, screw holes 47 in which bolts 14 with hexagon holes are threaded, and screw holes 48 in which bolts 12 with hexagon holes are threaded.

The prism housing portion 41 includes two walls 41a sandwiching the right-angle prism 61 therebetween. The right-angle prism 61 is fixed to the walls 41a by means of adhesive. The prism housing portion 42 includes two walls 42a sandwiching the right-angle prism 62 therebetween. The right-angle prism 62 is fixed to the walls 42a by means of adhesive.

The holder 40 surrounds at least a part of the optical path 10a from the white LED 72 to the RGB sensor 73 by means of the LED housing portion 43, the hole 45, the prism housing portion 41, the prism housing portion 42, the hole 46 and the RGB sensor housing portion 44.

The surface of the holder 40 is subjected to a treatment for preventing light reflection such as a black alumite treatment for matting.

The holder 40 supports the white LED 72 and the RGB sensor 73 via the circuit board 71. The holder 40 is provided with a substrate attaching portion 30a on which the circuit board 71 is attached via the space 13 and the bolts 14 with hexagon holes. Further, the holder 40 directly supports the gap forming member 60.

As shown in Fig. 12, the oil gap 60a of the gap forming member 60 is disposed on the optical path 10a from the white LED 72 to the RGB sensor 73.

The right-angle prisms 61, 62 transmit light emitted from the white LED 72. The right-angle prism 61 is provided with a light incident surface 61a on which light emitted from the white LED 72 is made incident, a light reflection surface 61b which reflects the light entered from the light incident surface 61a in a manner of bending the propagation direction of the light by 90 degrees, and a light emission surface 61c which emits the light reflected by the light reflection surface 61b. The right-angle prism 62 is provided with a light incident surface 62a on which light emitted from the light emission surface 61c of the right-angle prism 61 is made incident, a light reflection surface 62b which reflects the light entered from the light incident surface 62a in a manner of bending the propagation direction of the light by 90 degrees, and a light emission surface 62c which emits the light reflected by the light reflection surface 62b.

Each of the light incident surface 61a, the light reflection surface 61b and the light emission surface 61c of the right-angle prism 61 and the light incident surface 62a, the light reflection surface 62b and the light emission surface 62c of the right-angle prism 62 is optically polished. Further, each of the light reflection surface 61b of the right-angle prism 61 and the light reflection surface 62b of the right-angle prism 62 is provided with an aluminum deposition film. Furthermore, an $SiO_2$ film is formed on the aluminum deposition film in order to protect the aluminum deposition film which is low in a degree of hardness and an adhesive force.

The optical path 10a is bent by 90 degrees by the light reflection surface 61b of the right-angle prism 61 and also bent by 90 degrees by the light reflection surface 62b of the right-angle prism 62. That is, the optical path 10a is bent by 180 degrees by the gap forming member 60.

When a distance between the light emission surface 61c of the right-angle prism 61 and the light incident surface 62a of the right-angle prism 62, that is, the length of the oil gap 60a is too short, the contaminant within the lubricant 131a unlikely flows suitably through the oil gap 60a. Thus, the detection accuracy of the colors of the contaminant within the lubricant 131a degrades. On the other hand, when the length of the oil gap 60a is too long, the light emitted from the white LED 72 is excessively absorbed by the contaminant within the lubricant 131a within the oil gap 60a and hence unlikely reaches the RGB sensor 73. Thus, the detection accuracy of the colors of the contaminant within the lubricant 131a also degrades. Accordingly, preferably, the length of the oil gap 60a is set suitably so that the detection accuracy of the colors of the contaminant within the lubricant 131a becomes high. The length of the oil gap 60a is 1 mm, for example.

The white LED 72 is an electronic component which emits white light and constitutes a light emitting element according to this invention. As the white LED 72, NSPW500GS-K1 manufactured by Nichia Corporation, for example, may be employed.

The RGB sensor 73 is an electronic component which detects the colors of the received light and constitutes a color light reception element according to this invention. As the RGB sensor 73, S9032-02 manufactured by Hamamatsu Photonics K.K., for example, may be employed.

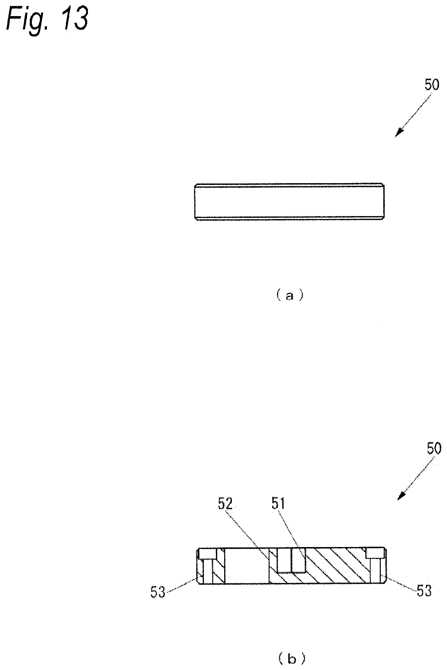

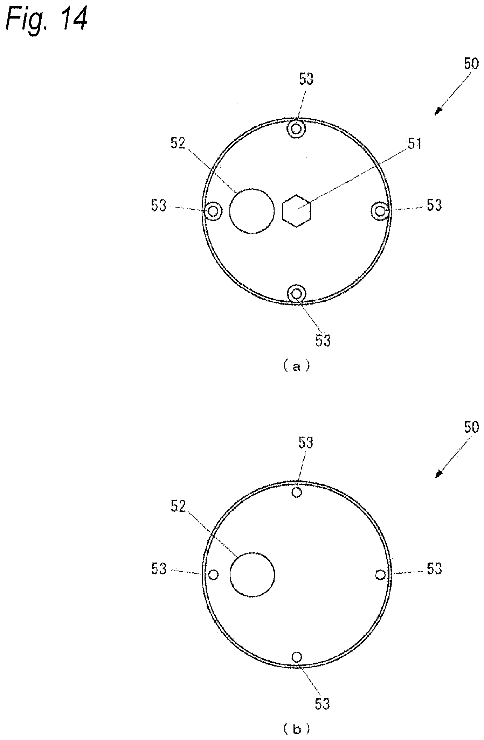

Fig. 13(a) is a front view of the holder cap 50. Fig. 13(b) is a front sectional view of the holder cap 50. Fig. 14(a) is a plan view of the holder cap 50. Fig. 14(b) is a bottom view of the holder cap 50.

As shown in Figs. 3 to 5(b) and Figs. 13(a) to 14(b), the holder cap 50 is provided with a tool contact portion 51 for contacting with a tool such as a hexagonal wrench at the time of rotating the supporting member 30 with respect to the housing 20. The tool contact portion 51 is a portion for receiving a rotational driving force of the supporting member 30 with respect to the housing 20 from the outside by a contact force, and constitutes a rotational driving force receiving portion according to this invention. The tool contact portion 51 is disposed at a position not contacting with the lubricant 131a when the housing 20 is fixed to the arm 113. Further, the holder cap 50 is provided with a hole 52 in which the waterproof connector 76 is inserted and holes 53 in which the bolts 12 with hexagon holes are inserted.

The surface of the holder cap 50 is subjected to the treatment for preventing light reflection such as the black alumite treatment for matting.

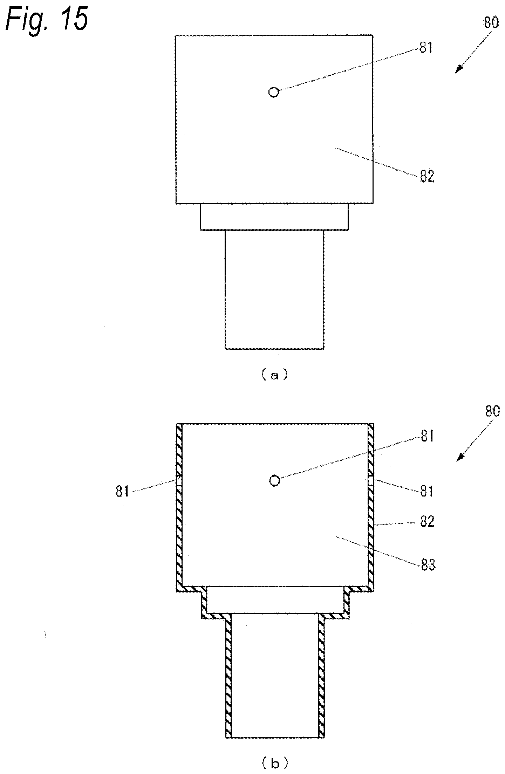

Fig. 15(a) is a front view of the shock absorbing member 80. Fig. 15(b) is a front sectional view of the shock absorbing member 80.

As shown in Fig. 15(a) and Fig. 15(b), the shock absorbing member 80 is provided with the hole 81 in which the bolts 11 with hexagon holes are inserted. The shock absorbing member 80 is a membrane like member having an approximately uniform thickness. The shock absorbing member 80 is disposed on the peripheral space of a substrate attaching portion 10b which is formed by surrounding the substrate attaching portion 30a of the holder 40 between the housing 20 and the holder 40. The shock absorbing member 80 includes the fixing member adhesion portion 82 which is a continuous portion surrounding the supporting member 30 and adheres to the housing 20 and the supporting member adhesion portion 83 which is a continuous portion surrounding the supporting member 30 and adheres to the supporting member 30. Meanwhile, the shock absorbing member 80 may be any material other than the soft rubber as long as it is a material capable of absorbing shocks. The shock absorbing member 80 is integrally formed.

Next, the assembling method of the lubricant deterioration sensor 139b will be explained. Although the explanation is made hereinafter as to the lubricant deterioration sensor 139b, the assembling method of each of the lubricant deterioration sensors such as the lubricant deterioration sensors 137a, 137b, and 139a other than the lubricant deterioration sensor 139b is substantially same.

First, adhesive is pasted on the surface plane of the prism housing portion 41 of the holder 40 to be made in contact with the light incident surface 61a of the right-angle prism 61, and also adhesive is pasted on the two surface planes of the right-angle prism 61 to be made in contact with the two walls 41a of the prism housing portion 41. Then, the right-angle prism 61 is fixed to the prism housing portion 41 by the adhesive. Further, adhesive is pasted on the surface plane of the prism housing portion 42 of the holder 40 to be made in contact with the light emission surface 62c of the right-angle prism 62, and also adhesive is pasted on the two surface planes of the right-angle prism 62 to be made in contact with the two walls 42a of the prism housing portion 42. Then, the right-angle prism 62 is fixed to the prism housing portion 42 by the adhesive. Furthermore, the white LED 72 is fixed to the LED housing portion 43 of the holder 40 by means of adhesive.

Then, the circuit board 71 which has the RGB sensor 73 and the connector 74 mounted thereon is fixed to the holder 40 by means of the bolts 14 with hexagon holes via the spacer 13, and the white LED 72 is fixed to the circuit board 71 by means of soldering.

Next, the connector 75 which is connected to the water proof connector 76 fixed to the holder cap 50 via the lead line 77 is connected to the connector 74 on the circuit board 71.

Next, the holder cap 50 is fixed to the holder 40 by means of the bolts 12 with hexagon holes. In this manner, the supporting member 30 to which the gap forming member 60 and the electronic component group 70 are attached is assembled.

Lastly, the supporting member 30 is fixed, by means of the bolts 11 with hexagon holes, to the holder housing portion 23 of the housing 20 attached with the O ring 15, and the shock absorbing member 80.

Next, the explanation will be made as to a method of mounting the lubricant deterioration sensor 139b to the arm 113. Although the explanation is made hereinafter as to the lubricant deterioration sensor 139b, the mounting method of each of the lubricant deterioration sensors such as the lubricant deterioration sensors 137a, 137b, and 139a other than the lubricant deterioration sensor 139b is substantially same.

First, the tool contact portion 22 of the housing 20 is grasped by a tool and the screw portion 21 of the housing 20 is inserted into the screw hole 113a of the arm 113. That is, the lubricant deterioration sensor 139b is fixed to the arm 113 by threading the screw portion into the screw hole.

Then, the connector of the external deice at the outside of the lubricant deterioration sensor 139b is connected to the water-proof connector 76.

Next, the operation of the industrial robot 100 will be explained.

First, the operation of the articular portion 130 will be explained. Although the explanation is made hereinafter as to the articular portion 130, the operation of each of the articular portions 120, 140 to 170 is substantially same.

When the output shaft of the motor 138 of the articular portion 130 rotates, the rotation speed of the motor 138 is reduced by the reducer 131, whereby the arm 113 fixed to the supporting body 134 of the reducer 131 is moved by the rotation force of the motor with respect to the arm 112 fixed to the case 133 of the reducer 131.

Next, the operation of the lubricant deterioration sensor 139b will be explained. Although the explanation is made hereinafter as to the lubricant deterioration sensor 139b, the operation of each of the lubricant deterioration sensors such as the lubricant deterioration sensors 137a, 137b, and 139a other than the lubricant deterioration sensor 139b is substantially same.

The lubricant deterioration sensor 139b emits white light from the white LED 72 in response to electric power supplied from the external device via the water-proof connector 76.

Then, the lubricant deterioration sensor 139b outputs light amounts of respective colors RGB of the light received by the RGB sensor 73 as an electric signal to the external device via the water-proof connector 76.

The lubricant deterioration sensor 139b may additionally mount another sensor other than the RGB sensor 73. For example, in the lubricant deterioration sensor 139b, when a temperature sensor for detecting the temperature of the lubricant 131a is contained in the electronic component group 70, the temperature detected by the temperature sensor can also be output as an electric signal to the external device via the water-proof connector 76.

Next, the explanation will be made as to a method of adjusting the direction of the opening 60b of the oil gap 60a of the lubricant deterioration sensor 139b. Although the explanation is made hereinafter as to the lubricant deterioration sensor 139b, the adjusting method of each of the lubricant deterioration sensors such as the lubricant deterioration sensors 137a, 137b, and 139a other than the lubricant deterioration sensor 139b is substantially same.

The external device of the lubricant deterioration sensor 139b can specify the kinds and amounts of the contaminant within the lubricant 131a of the reducer 131 based on the colors detected by the RGB sensor 73. That is, the lubricant deterioration sensor 139b can detect the deterioration degree of the lubricant 131a by detecting the colors of the contaminant within the lubricant 131a.

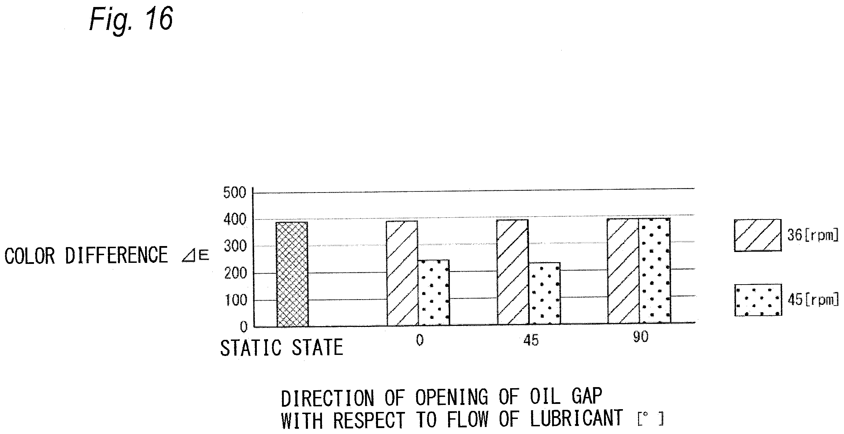

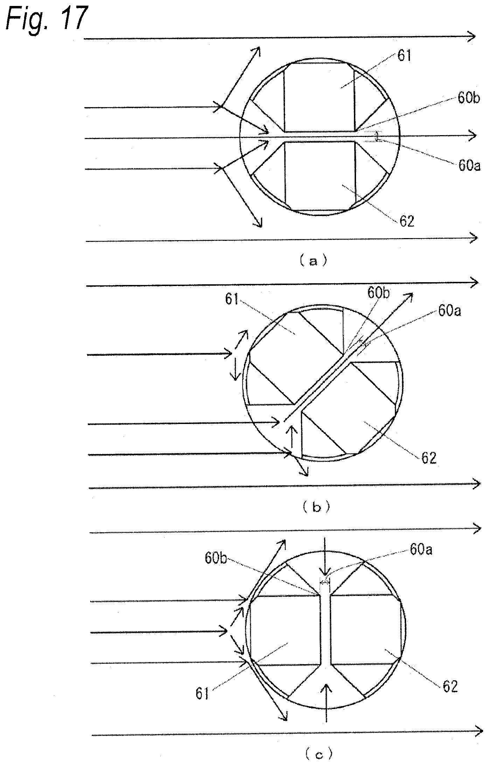

Fig. 16 is a diagram showing an example of the relation between the direction of the opening 60b of the oil gap 60a with respect to the flow of the lubricant 131a and a color difference ΔE of the colors detected by the RGB sensor 73 with respect to black. Fig. 17(a) is a diagram showing a state that the direction of the opening 60b of the oil gap 60a with respect to the flow of the lubricant 131a is 0 degree. Fig. 17(b) is a diagram showing a state that the direction of the opening 60b of the oil gap 60a with respect to the flow of the lubricant 131a is 45 degrees. Fig. 17(c) is a diagram showing a state that the direction of the opening 60b of the oil gap 60a with respect to the flow of the lubricant 131a is 90 degrees.

The color difference ΔE of the colors detected by the RGB sensor 73 with respect to black can be calculated by an expression shown by the following Math. 1 by using the respective values of the colors RGB detected by the RGB sensor 73.

[Formula 1]

$$\Delta E = \sqrt{R^2 + G^2 + B^2}$$

In the experiment where the relation shown in Fig. 16 was derived, new oil with a low deterioration degree was used as the lubricant 131a.

Further, in Fig. 16, a "static state" represents a time period where the flow of the lubricant 131a stops. When the flow of the lubricant 131a stops, the direction of the opening 60b of the oil gap 60a with respect to the flow of the lubricant 131a does not influence on the ΔE of the colors detected by the RGB sensor 73 with respect to black. Thus, the ΔE of the colors detected by the RGB sensor 73 with respect to black in the "static state" becomes a determination criterion of the relation between the direction of the opening 60b of the oil gap 60a with respect to the flow of the lubricant 131a and the color difference ΔE of the colors detected by the RGB sensor 73 with respect to black.

Further, in Fig. 16, each of 36 [rpm] and 45 [rpm] represents the rotation speed of the arm 113 with respect to the arm 112 as a revolution number per one minute. Since the lubricant deterioration sensor 139b is attached to the arm 113, this sensor moves within the lubricant 131a in accordance with the rotation of the arm 113 with respect to the arm 112. In other words, each of 36 [rpm] and 45 [rpm] indirectly represents the speed of the flow of the lubricant 131a with respect to the lubricant deterioration sensor 139b.

In Figs. 17(a) to 17(c), arrows other than those representing the oil gap 60a represent the flow of the lubricant 131a.

The detection accuracy of the deterioration of the lubricant 131a can be determined by the color difference ΔE of the colors detected by the RGB sensor 73 with respect to black. In other words, in Fig. 16, the detection accuracy of the deterioration of the lubricant 131a degrades in a case that the rotation speed of the arm 113 with respect to the arm 112 is 45 [rpm] and the direction of the opening 60b of the oil gap 60a with respect to the flow of the lubricant 131a is 0 degree or 45 degrees. In this manner, the detection accuracy of the deterioration of the lubricant 131a degrades depending on the direction of the opening 60b of the oil gap 60a with respect to the flow of the lubricant 131a.

The lubricant deterioration sensor 139b is configured to be adjustable in the direction of the opening 60b of the oil gap 60a.

First, each of the bolts 11 with hexagon holes is loosened by a tool inserted into the tool contact portion 11a so that the supporting member 30 becomes rotatable with respect to the housing 20.

Then, in a state that the rotation of the housing 20 with respect to the arm 113 is prevented by grasping the tool contact portion 22 of the housing 20 by the tool, the supporting member 30 is rotated with respect to the housing 20 by the tool inserted into the tool contact portion 51. The direction of the opening 60b of the oil gap 60a changes in accordance with the rotation of the supporting member 30 with respect to the housing 20.

Lastly, each of the bolts 11 with hexagon holes is fastened by the tool inserted into the tool contact portion 11a so that the rotation of the supporting member 30 becomes impossible with respect to the housing 20.

As explained above, the RGB sensor 73 detects colors with respect to the light having wavelengths not absorbed by the contaminant within the lubricant 131a at the oil gap 60a, among the white light emitted by the white LED 72. Thus, each of the lubricant deterioration sensors such as the lubricant deterioration sensor 139b can immediately detect the colors of the contaminant within the lubricant 131a of the reducer 131. That is, each of the lubricant deterioration sensors can immediately specify the kinds and amounts of the contaminant within the lubricant 131a of the reducer 131 based on the colors detected by the RGB sensor 73, by using the external device such as a computer. Each of the lubricant deterioration sensors may be configured that the electronic component group 70 contains an electronic component which specifies the kinds and amounts of the contaminant within the lubricant based on the colors detected by the RGB sensor 73.

Generally, in the industrial robot, accuracy of the locus of the arm etc. largely depends on the performance of the reducer used at the articular portion. Thus, it is important to suitably exchange the reducer for the industrial robot for new one when the performance of the reducer degrades. However, in the case of exchanging the reducer for the industrial robot, it is required to stop the industrial robot provided with this reducer and a production line installing the industrial robot. Thus, in order to grasp the exchange time of the reducer for the industrial robot, it is very important to suitably predict a failure of the reducer for the industrial robot. In this respect, as described above, each of the lubricant deterioration sensors of the industrial robot 100 enables to immediately specify the kinds and amounts of the contaminant within the lubricant 131a of the reducer 131 based on the colors detected by the RGB sensor 73, by using the external device such as a computer. Thus, the industrial robot 100 and each of the reducers of the industrial robot 100 enables to perform immediate prediction of a failure.

In addition, in a case where the housing 20 is subjected to shocks from the arm 113, each of the lubricant deterioration sensors such as the lubricant deterioration sensor 139b enables suppression of a possibility of damaging the electronic component mounted on the circuit board 71 which is attached to the substrate attaching portion 30a due to the shocks since the shocks transferred to the substrate attaching portion 30a of the supporting member 30 from the housing 20 can be absorbed by the shock absorbing member 80 which is disposed between the housing 20 and the supporting member 30. Accordingly, each of the lubricant deterioration sensors such as the lubricant deterioration sensor 139b enables suppression of a possibility of the failure in a case where the housing 20 is subjected to shocks from the arm 113. In other words, the respective reducers of the industrial robot 100 and the industrial robot 100 enables the suppression of the failure of the lubricant deterioration sensor which enables immediate specification of the kinds and amounts of contaminant within the lubricant 131a, thus it is possible to maintain for a long time the accuracy of the immediate prediction as to a failure.

In addition, in a case where the housing 20 is subjected to shocks from the arm 113, each of the lubricant deterioration sensors such as the lubricant deterioration sensor 139b enables suppression of a possibility of changing a positional relationship of the white LED 72 and the RGB sensor 73 mounted on the circuit board 71 which is attached to the substrate attaching portion 30a due to the shocks since the shocks transferred to the substrate attaching portion 30a of the supporting member 30 from the housing 20 can be absorbed by the shock absorbing member 80 disposed between the housing 20 and the supporting member 30. Accordingly, each of the lubricant deterioration sensors such as the lubricant deterioration sensor 139b enables suppression of a possibility of the degradation of the detection accuracy of the deterioration of the lubricant 131a of the arm 113 in a case where the housing 20 is subjected to shocks from the arm 113.

In addition, since the industrial robot 100 applies the acceleration to each of the lubricant deterioration sensors such as the lubricant deterioration sensor 139b in accordance with an operation of the industrial robot 100 itself, applies a vibration to the lubricant deterioration sensor by the vibration of the industrial robot 100 itself, and applies a vibration to the lubricant deterioration sensor by the vibration of each motor such as a motor 138, the lubricant deterioration sensor is subjected to shocks generated due to vibration and acceleration for a long time. Accordingly, the industrial robot 100 and the reducer for the industrial robot 100 can suppress a possibility of the failure of the lubricant deterioration sensor in a case where the lubricant deterioration sensor is subjected to shocks from the machine body and a possibility of the degradation of the detection accuracy of deterioration of the lubricant of the machine body by the lubricant deterioration sensor.

In addition, since the shock absorbing member 80 includes a fixing member adhesion portion 82 and a supporting member adhesion portion 83, each of the lubricant deterioration sensors such as the lubricant deterioration sensor 139b enables not only to suppress the leakage of the lubricant 131a from between the housing 20 and the supporting member 30 by the shock absorbing member 80 but also absorb the shocks transferred to the supporting member 30 from the housing 20 by the shock absorbing member 80.

In each of the lubricant deterioration sensors such as the lubricant deterioration sensor 139b, the supporting member 30 is disposed on the screw portion 21 in a manner that a part of the optical path 10a is disposed on the inner side of the screw portion 21 and a portion of the shock absorbing member 80 is disposed between the screw portion 21 and the supporting member 30. Thus, for example, in a case where a screw hole such as the screw hole 113a is formed to be small as compared with the screw portion 21, even if the screw portion 21 of the housing 20 is made in contact on the outer side thereof with the screw hole of the machine body such as the screw hole 113a of the arm 113 and is deformed toward the inner side, the deformation of the screw portion 21 is hardly transmitted to the supporting member 30 by the shock absorbing member 80 which is disposed between the screw 21 and the supporting member 30. Accordingly, the change of the optical path 10a due to the deformation of the supporting member 30 hardly occurs. As a result, in the case where each of the lubricant deterioration sensors such as the lubricant deterioration sensor 139b is fixed to the machine body by means of the screw portion 21, the detection accuracy of the deterioration of the lubricant 131a of the machine body can be suppressed from degrading.

In each of the lubricant deterioration sensors such as the lubricant deterioration sensor 139b, the screw portion 21 likely deforms toward the inner side thereof when the screw portion 21 is threaded into the screw hole of the machine body and made in contact with the machine body. Thus, in the case of fixing the screw portion 21 to the machine body, the detection accuracy of the deterioration of the lubricant of the machine body can be suppressed efficiently from degrading.

In a case where the housing 20 includes a component other than the screw portion 21 to be fixed to the machine body such as the arm 113, the screw portion 21 shown in Fig. 3 may simply be a contact portion that contacts with the machine body on the outer side thereof. For example, in the housing 20, the screw portion 21 may simply be a cylindrical portion having no screw. In each of the lubricant deterioration sensors such as the lubricant deterioration sensor 139b, in the case where the contact portion acts as a portion to be inserted into the hole of the machine body, the contact portion likely deforms toward the inside when the contact portion is inserted into the hole of the machine body and made in contact with the machine body. Thus, like the configuration where the contact portion is the screw portion 21, in the case where the contact portion contacts with the machine body, the detection accuracy of the deterioration of the lubricant of the machine body can be suppressed efficiently from degrading. In each of the lubricant deterioration sensors such as the lubricant deterioration sensor 139b, even in the configuration that the contact portion is made in contact with the machine body without being inserted into the hole of the machine body, even if the contact portion is made in contact on the outer side thereof with the machine body and is deformed toward the inner side, the deformation of the contact portion is hardly transmitted to the supporting member 30 by the shock absorbing member 80 which is disposed between the contact portion and the supporting member 30. Thus, in the case where the contact portion contacts with the machine body, the detection accuracy of the deterioration of the lubricant of the machine body can be suppressed from degrading.

To the lubricant 131a, there is sometimes added various kinds of additive such as friction reducing agent like organic molybdenum such as MoDTC or MoDTP for reducing the friction of a friction surface, extreme-pressure additive such as SP-based additive for improving extreme-pressure lubricity representing the performance for suppressing the seizure of the friction surface, and dispersing agent such as calcium sulfonate for suppressing the generation and adhesion of sludge. This additive is separated from the lubricant 131a in accordance with the deterioration of the lubricant 131a in such a manner that the additive adheres to, binds with or settles on the metal surface of the industrial robot 100 and the reducer. Each of the lubricant deterioration sensors can specify, based on the detected colors, not only an amount of ion powder within the lubricant 131a but also a deterioration degree of the base oil and increase of the contaminant such as sludge due to the reduction of various kinds of additive added to the lubricant 131a. Thus, each of the industrial robot 100 and the reducers of the industrial robot 100 can improve the prediction accuracy of a failure as compared with a technique where a failure of the reducer is predicted only based on a density of iron powder.

Further, in each of the lubricant deterioration sensors such as the lubricant deterioration sensor 139b, the housing 20 supports the supporting member 30 so as to be rotatable so that the direction of the opening 60b of the oil gap 60a changes when the supporting member 30 rotates. Thus, in a case where the housing 20 is fixed to the industrial robot 100, the direction of the opening 60b of the oil gap 60a at the time of fixing the housing 20 to the industrial robot 100 can be adjusted so that the detection accuracy of the deterioration of the lubricant 131a of the industrial robot 100 becomes high. As a result, each of the industrial robot 100 and the reducers of the industrial robot 100 enables to predict a failure with a high accuracy.

Further, in each of the lubricant deterioration sensors such as the lubricant deterioration sensor 139b, the supporting member 30 includes the tool contact portion 51 at a position not contacting with the lubricant 131a when the housing 20 is fixed to the industrial robot 100. The tool contact portion is a portion which receives the rotational driving force with respect to the housing 20 from the outside by the contact force. Thus, in each of the lubricant deterioration sensors, after the housing 20 is fixed to the industrial robot 100, the direction of the opening 60b of the oil gap 60a at the time of fixing the housing 20 to the industrial robot 100 can be adjusted so that the detection accuracy of the deterioration of the lubricant 131a of the industrial robot 100 becomes high.

Furthermore, in each of the lubricant deterioration sensors such as the lubricant deterioration sensor 139b, each of the bolts 11 ith hexagon holes includes the tool contact portion 11 at the position not contacting with the lubricant 131a when the housing 20 is fixed to the industrial robot 100. Each of the bolts with hexagon holes is configured so as to prevent the rotation of the supporting member 30 with respect to the housing 20 by contacting with both the supporting member 30 and the housing 20. The tool contact portion is a portion which receives the driving force for contacting with both the supporting member 30 and the housing 20 from the outside by the contact force. Thus, in each of the lubricant deterioration sensors, after the housing 20 is fixed to the industrial robot 100, the direction of the opening 60b of the oil gap 60a at the time of fixing the housing 20 to the industrial robot 100 can be fixed so that the detection accuracy of the deterioration of the lubricant 131a of the industrial robot 100 becomes high.

Furthermore, in each of the lubricant deterioration sensors, since the light emitting element is the white LED for emitting white light, the sensor can be miniaturized as compared with a configuration where the light emitting element is a lamp other than an LED, for example. Thus, each of the industrial robot 100 and the reducers of the industrial robot 100 can be miniaturized. The light emitting element according to this invention may be one other than the white LED. For example, the light emitting element may be a lamp other than an LED. Further, the light emitting element may be configured to include a red LED or a red lamp other than an LED, a green LED or a green lamp other than an LED and a blue LED or a blue lamp other than an LED, to thereby emit white light by composing light of respective colors emitted from these LEDs or these lamps other than LEDs.

Furthermore, in each of the lubricant deterioration sensors, the gap forming member 60 is provided with the light reflection surfaces 61b, 62b for bending the optical path 10a. Thus, as compared with the configuration where the optical path 10a from the white LED 72 to the RGB sensor 73 is straight, the entire configuration can be miniaturized by disposing the white LED 72 and the RGB sensor 73 close to each other. Furthermore, in each of the lubricant deterioration sensors, the gap forming member 60 has a function of bending the optical path 10a as well as a function of forming the oil gap 60a. Thus, the number of the components can be reduced as compared with a configuration where a member for bending the optical path 10a is separately provided in place of the gap forming member 60. As a result, each of the industrial robot 100 and the reducers of the industrial robot 100 can be miniaturized and also reduce the number of the components.

In particular, each of the lubricant deterioration sensors is configured in a manner that the gap forming member 60 is formed by the two right-angle prisms 61, 62 respectively provided with the light reflection surfaces 61b, 62b each for bending the optical path 10a by 90 degrees, and that the optical path 10a is bent by 180 degrees by the light reflection surfaces 61b, 62b of the two right-angle prisms 61, 62 and the oil gap 60a is formed between the two right-angle prisms 61, 62. Thus, the sensor can be miniaturized with the simple configuration having a small number of the components. As a result, each of the industrial robot 100 and the reducers of the industrial robot 100 can be miniaturized with the simple configuration having the small number of the components.

Furthermore, each of the lubricant deterioration sensors is configured in a manner that the sensor has the holder 40 surrounding at least a part of the optical path 10a and the surface of the holder 40 is subjected to the treatment for preventing light reflection. Thus, the RGB sensor 73 can be prevented from receiving unnecessary reflection light. As a result, as compared with a configuration where the RGB sensor 73 receives unnecessary reflection light, each of the lubricant deterioration sensors can improve the detection accuracy of the colors of the contaminant within the lubricant 131a. Accordingly, the industrial robot 100 and each of the reducers of the industrial robot 100 can improve the prediction accuracy of a failure.

Furthermore, in each of the lubricant deterioration sensors, an oil repelling treatment may be performed on the surface planes of the gap forming member 60 forming the oil gap 60a, that is, the light emission surface 61c of the right-angle prism 61 and the light incident surface 62a of the right-angle prism 62. In each of the lubricant deterioration sensors, when each of the light emission surface 61c of the right-angle prism 61 and the light incident surface 62a of the right-angle prism 62 is subjected to the oil repelling treatment, the lubricant 131a flows through the oil gap 60a easily. Thus, as compared with a configuration where the lubricant 131a likely remains at the oil gap 60a, the detection accuracy of the colors of the contaminant within the lubricant 131a can be improved. Furthermore, in each of the lubricant deterioration sensors, when each of the light emission surface 61c of the right-angle prism 61 and the light incident surface 62a of the right-angle prism 62 is subjected to the oil repelling treatment, dirt is unlikely adhered to each of the light emission surface 61c of the right-angle prism 61 and the light incident surface 62a of the right-angle prism 62. Thus, the degradation of the detection accuracy of the colors of the contaminant within the lubricant 131a due to the adhesion of dirt can be suppressed. Accordingly, each of the industrial robot 100 and the reducers of the industrial robot 100 can improve the prediction accuracy of a failure.

The prediction accuracy of a failure of the reducer can be improved by jointly using the lubricant deterioration sensor according to this invention, the temperature sensor for measuring the temperature of the lubricant and a monitoring mechanism for a current value of the motor etc.

Although each of the right-angle prisms 61, 62 of the gap forming member 60 is made of glass in this embodiment, each of them may be formed by material other than glass such as silicon resin. When each of the prisms 61, 62 is formed by silicon resin, dirt can be unlikely adhered to the surface planes thereof forming the oil gap 60a.

Although the gap forming member 60 is configured by the two right-angle prisms 61, 62 in this embodiment, the gap forming member may be configured by three or more prisms.

In each of the lubricant deterioration sensors, the white LED 72 and the RGB sensor 73 may be configured in an arrangement other than that explained in this embodiment. For example, in each of the lubricant deterioration sensors, the optical path 10a from the white LED 72 to the RGB sensor 73 may be straight.

Further, in each of the lubricant deterioration sensors, the optical path 10a may be bent by employing a configuration other than the right-angle prisms.

In each of the lubricant deterioration sensors, for example, a battery such as a battery cell may be used as an electric power supply means, and a wireless communication may be employed as a means for outputting the detection result to the external device.

The configuration of an industrial robot as a machine according to the second embodiment will be explained.

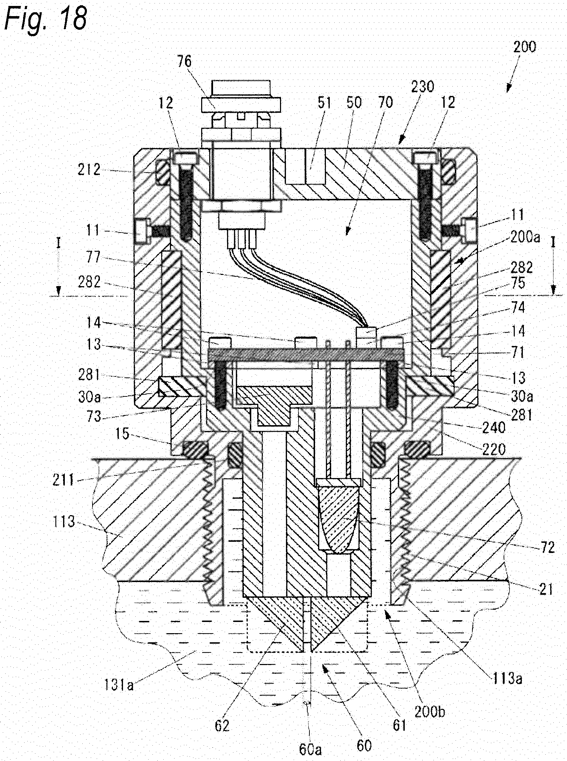

The configuration of the industrial robot according to the embodiment is the same as the configuration of the industrial robot 100 (refer to Fig. 1) according to the first embodiment, in which the configuration is to have a lubricant deterioration sensor 200 shown in Fig. 18 instead of each of the lubricant deterioration sensors such as the lubricant deterioration sensor 139b (refer to Fig. 3). Accordingly, regarding the configuration other than the lubricant deterioration sensor 200 of the configuration of the industrial robot according to this embodiment, components same as those of the industrial robot 100 are referred to by the same symbols as those of the industrial robot 100, with detailed explanation thereof being omitted.

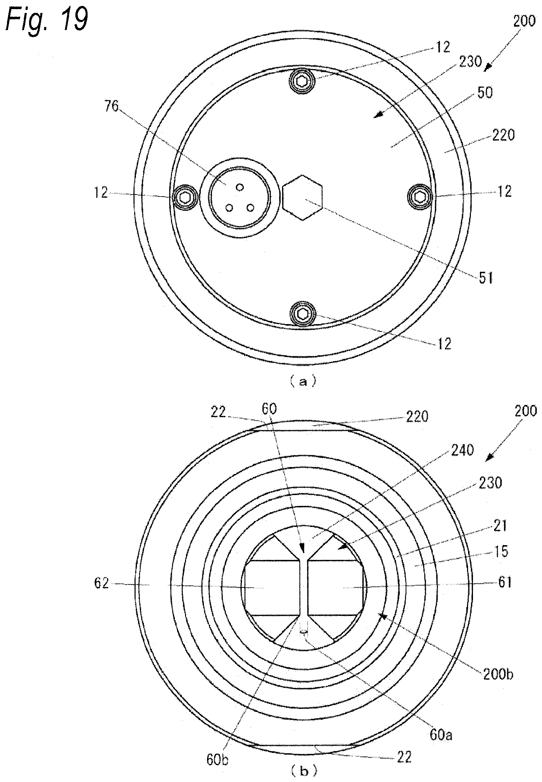

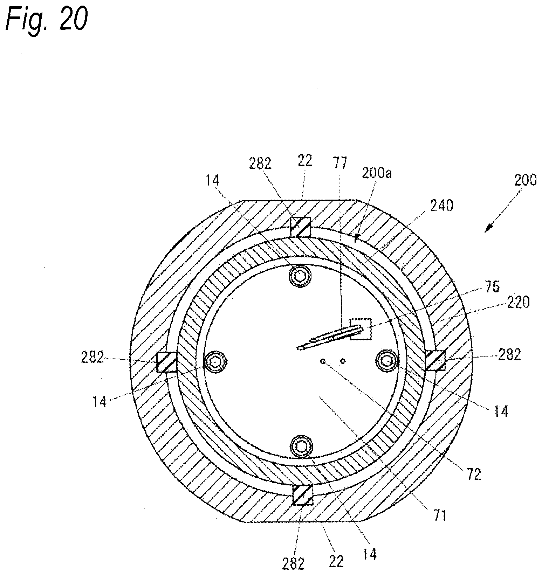

Fig. 18 is a front sectional view of the lubricant deterioration sensor 200 in a state where the lubricant deterioration sensor is attached to the arm 113. Fig. 19(a) is a plan view of the lubricant deterioration sensor 200. Fig. 19(b) is a bottom view of the lubricant deterioration sensor 200. Fig. 20 is a cross-sectional view taken along line I-I of Fig. 18.

As shown in Fig. 18 to Fig. 20, the lubricant deterioration sensor 200 is configured to include a housing 220 made of an aluminum alloy, in which the lubricant deterioration sensor 139b (refer to Fig. 4) supports each component of the lubricant deterioration sensor 200, a supporting member 230 supporting the gap forming member 60, and shock absorbing members 281 and 282 made of soft rubber which are capable of absorbing shocks and disposed between the housing 220 and the supporting member 230 instead of the housing 20 (refer to Fig. 4), the supporting member 30 (refer to Fig. 4), and the shock absorbing member 80 (refer to Fig. 4), and also to newly include O rings 211 and 212 in which the lubricant deterioration sensor 139b is disposed between the housing 220 and the supporting member 230.

The supporting member 230 is configured that the supporting member 30 (refer to Fig. 4) includes the holder 240 made of an aluminum alloy instead of a holder 40 (refer to Fig. 4).

The shock absorbing members 281 and 282 are in a rod shape of which the sectional shape is a square. The shock absorbing members 281 and 282 are disposed in the peripheral space of a substrate attaching portion 200a which is formed by surrounding the substrate attaching portion 30a of the holder 240 between the housing 220 and the holder 240. Meanwhile, the shock absorbing members 281 and 282 may be formed of any material other than the soft rubber as long as it is a material capable of absorbing shocks.

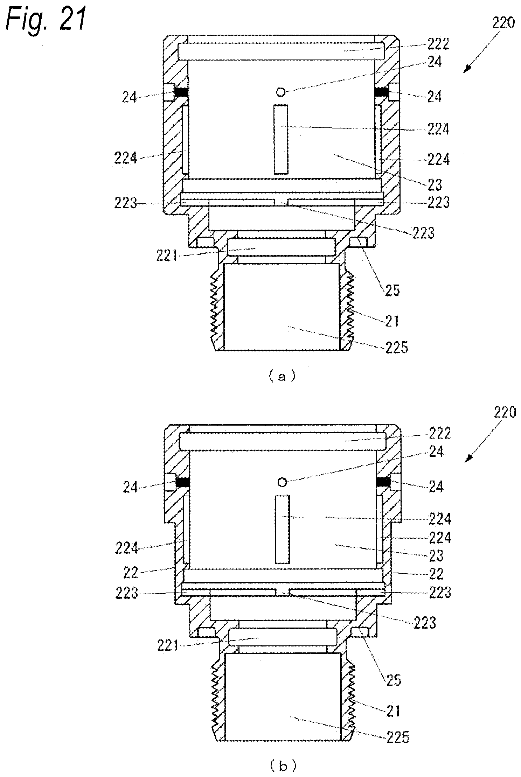

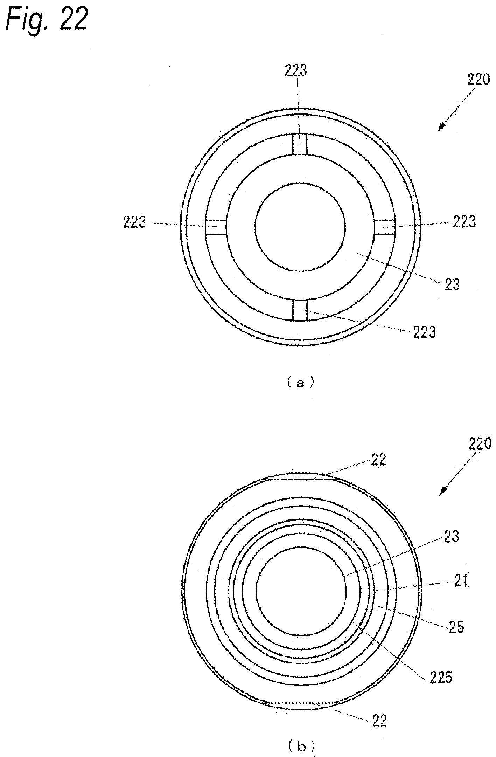

Fig. 21 (a) is a front sectional view of a housing 220. Fig. 21 (b) is a side cross-sectional view of the housing 220. Fig. 22(a) is a plan view of the housing 220. Fig. 22(b) is a bottom view of the housing 220.

As shown in Fig. 21 (a) to Fig. 22(b), the housing 220 includes the housing 20 (refer to Fig. 6(a) and Fig. 6(b)) is configured to include a groove 221 into which an O ring 211 is fit, a groove 222 into which an O ring 212 is fit, four grooves 223 into which the shock absorbing member 281 is fit, four grooves 224 into which the shock absorbing member 282 is fit, and an opening 225 which forms a gap 200b (refer to Fig. 18) between a screw portion 21 and a supporting member 230.

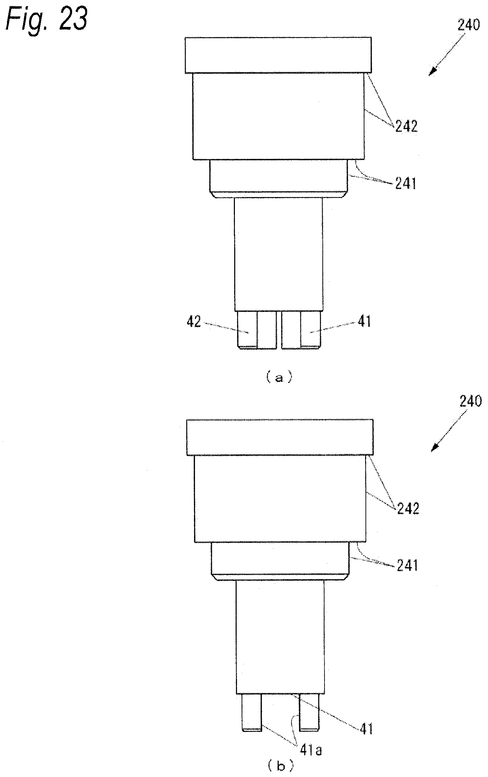

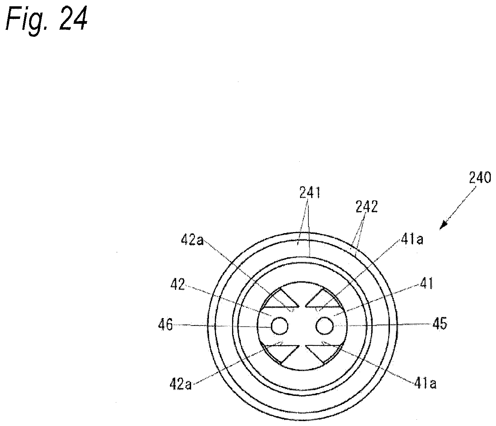

Fig. 23(a) is a front view of a holder 240. Fig. 23(b) is a side view of the holder 240. Fig. 24 is a bottom view of the holder 240.

As shown in Fig. 23(a) to Fig. 24, the holder 240 has the same configuration as that of the holder 40 (refer to Fig. 9), which includes the shock absorbing member contact portion 241 that comes in contact with the shock absorbing member 281, and the shock absorbing member contact portion 242 that comes in contact with the shock absorbing member 282.

The holder 240 is disposed on the inner side of the screw portion 21 far from the screw portion 21 so that a portion of the optical path 10a is disposed on the inner side of the screw portion 21 of the housing 220, that is, the gap 200b is formed between the screw portion 21 of the housing 220 and the holder 240 (refer to Fig. 18).

Next, an assembling method of a lubricant deterioration sensor 200 will be explained.

First, similar to the first embodiment, the supporting member 230 to which the gap forming member 60 and the electronic component group 70 are attached is assembled.

Then, the supporting member 230 is fixed to the holder housing portion 23 of the housing 220 to which the O ring 15, the O ring 211, the O ring 212, the shock absorbing member 281, and the shock absorbing member 282 are attached, by using the bolts 11 with hexagon holes.

As described above, the lubricant deterioration sensor 200 enables absorption of the shocks transferred to the substrate attaching portion 30a of the support member 230 from the housing 220 by the shock absorbing members 281 and 282 which are disposed between the housing 220 and the supporting member 230 when the housing 220 is subjected to shocks from the arm 113, and thus it is possible to suppress a possibility of damaging an electronic component mounted on the circuit board 71 which is attached to the substrate attaching portion 30a due to the shocks. Therefore, the lubricant deterioration sensor 200 enables suppression of a failure occurring when the housing 220 is subjected to shocks from the arm 113.

In addition, the lubricant deterioration sensor 200 enables absorption of the shocks transferred to the substrate attaching portion 30a of the support member 230 from the housing 220 by the shock absorbing members 281 and 282 which are disposed between the housing 220 and the supporting member 230 when the housing 220 is subjected to shocks from the arm 113, and thus it is possible to suppress a possibility of changing a positional relationship of the white LED 72 and RGB sensor 73 which are mounted on the circuit board 71 attached on the substrate attaching portion 30a by the shocks. Accordingly, when the housing 220 is subjected to shocks from the arm 113, the lubricant deterioration sensor 200 enables suppression of degrading the detection accuracy of the deterioration of the lubricant 131a of the arm 113.

As for the lubricant deterioration sensor 200, since the supporting member 230 is disposed on the inner side of the screw portion 21 far from the screw portion 21 so that a portion of the optical path 10a is disposed on the inner side of the screw portion 21, for example, when the screw hole such as the screw hole 113a is formed to be small as compared with the screw portion 21, the deformation of the screw portion 21 is unlikely to be transferred to the supporting member 230 disposed far from the screw portion 21, and thus the change of the optical path 10a due to the deformation of the supporting member 230 does not easily occur even in a case where the screw portion 21 of the housing 220 comes in contact with the outside of the screw hole of the machine body such as the screw hole 113a of the arm 113 and deforms to the inside. Therefore, the detection accuracy of the deterioration of the lubricant 131a of the machine body can be suppressed from degrading when the lubricant deterioration sensor 200 is fixed to the machine body by the screw portion 21.

Since an effect other than the above described effects among the effects of the lubricant deterioration sensor 200 is the same as the effect of the lubricant deterioration sensor 139b according to the first embodiment, the description is omitted.

The configuration of an industrial robot as a machine according to the third embodiment will be explained.

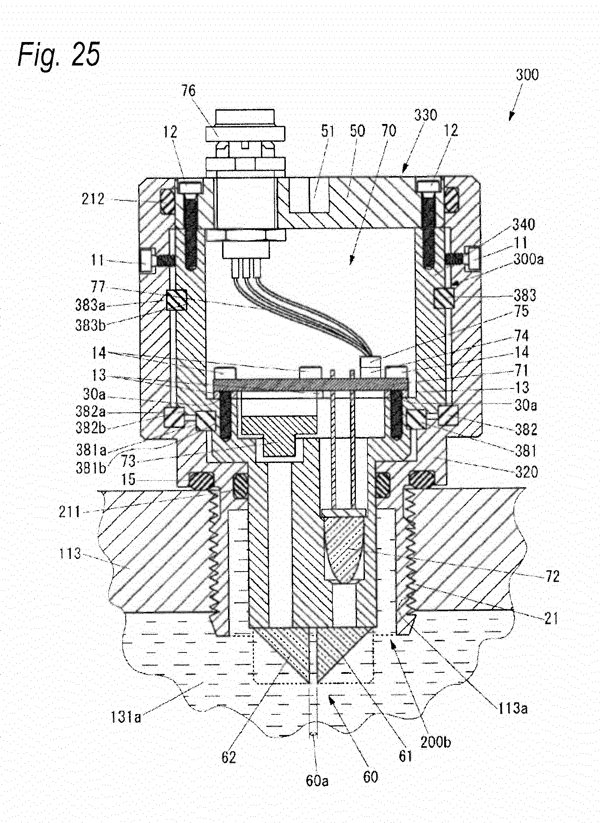

The configuration of the industrial robot according to the embodiment is the same as that of the industrial robot according to the second embodiment except that a lubricant deterioration sensor 300 shown in Fig. 25 is provided instead of the lubricant deterioration sensor 200 (refer to Fig. 18). Accordingly, of the components of the industrial robot as according to the embodiment, components other than the lubricant deterioration sensor 300 have the same reference numerals as the components of the industrial robot as a machine according to the second embodiment and detailed description is omitted.

Fig. 25 is a front sectional view of the lubricant deterioration sensor 300 in a state of being attached to arm 113.

As shown in Fig. 25, the configuration of the lubricant deterioration sensor 300 is the same as that of the lubricant deterioration sensor 200 (refer to Fig. 18) which includes a housing 320 which is made of an aluminum alloy and supports the respective components of the lubricant deterioration sensor 300, the supporting member 330 supporting the gap forming member 60, shock absorbing members 381, 382, and 383 which are made of soft rubber which absorbs shocks and disposed between the housing 320 and the supporting member 330 instead of the housing 220 (refer to Fig. 18), the supporting member 230 (refer to Fig. 18), and the shock absorbing members 281 and 282 (refer to Fig. 18).

The supporting member 330 is configured that the supporting member 230 (refer to Fig. 18) includes the holder 340 made of an aluminum alloy instead of a holder 240 (refer to Fig. 18).

The shock absorbing members 381, 382, and 383 are in an annular shape of which the sectional shape is a square. The shock absorbing members 381, 382, and 383 are disposed in the peripheral space of substrate attaching portion 300a which is formed by surrounding the substrate attaching portion 30a of the holder 340 between the housing 320 and holder 340. Each of the shock absorbing members 381, 382, and 383 includes each of the fixing member adhesion portions 381a, 382a, and 383a which is a continuous portion surrounding the supporting member 330 and adheres to the housing 320 and each of the supporting member adhesion portions 381b, 382b, and 383b which is a continuous portion surrounding the supporting member 330 and adheres to the supporting member 330. Meanwhile, the shock absorbing members 381, 382, and 383 may be any material other than the soft rubber as long as it is a material capable of absorbing shocks.

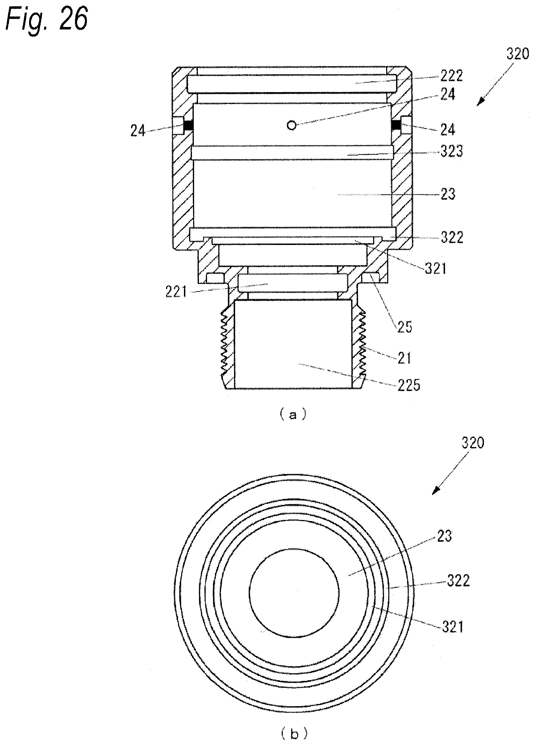

Fig. 26(a) is a front sectional view of the housing 320. Fig. 26(b) is a plan view of the housing 320.

As shown in Fig. 26(a) and Fig. 26(b), the housing 320 is configured to include an annular groove 321 into which the shock absorbing member 381 is fit, an annular groove 322 into which the shock absorbing member 382 is fit, and an annular groove 323 into which the shock absorbing member 383 is fit instead of the groove 223 (refer to Fig. 21 (a) and Fig. 21(b)) and the groove 224 (refer to Fig. 21 (a) and Fig. 21 (b)) in the housing 220 (refer to Fig. 21 (a) and Fig. 21 (b)).

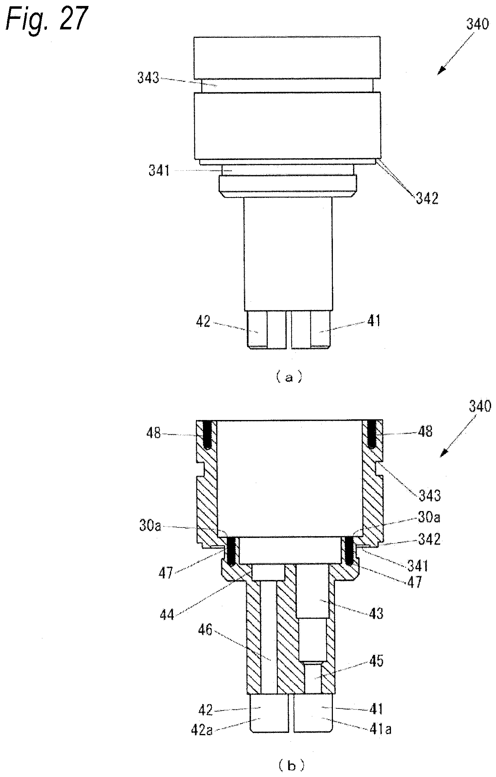

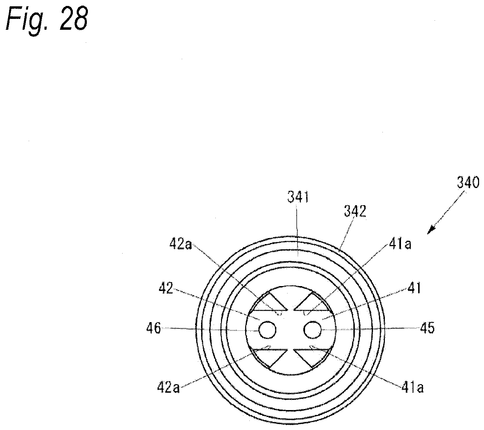

Fig. 27(a) is a front view of a holder 340. Fig. 27(b) is a front sectional view of the holder 340. Fig. 28 is a bottom view of the holder 340.

As shown in Fig. 27(a) to Fig. 28, the configuration of the holder 340 is the same as that of the holder 240 (refer to Fig. 23(a) and Fig. 23(b)) which includes an annular groove 341 into which the shock absorbing member 381 is fit, an annular groove 342 into which the shock absorbing member 382 is fit, and an annular groove 343 into which the shock absorbing member 383 is fit, while not including the shock absorbing member contact portion 241 (refer to Fig. 23(a) and Fig. 23(b)) and the shock absorbing member contact portion 242 (refer to Fig. 23(a) and Fig. 23(b)).

Next, an assembling method of the lubricant deterioration sensor 300 will be explained.

First, similar to the first embodiment, the supporting member 330 to which the gap forming member 60 and the electronic component group 70 are attached is assembled.

Then, supporting member 330 to which the shock absorbing member 381 and the shock absorbing member 383 are attached is fixed to the holder housing portion 23 of the housing 320 to which the O ring 15, the O ring 211, the O ring 212, and the shock absorbing member 382 are attached, by using the bolts 11 with hexagon holes.

As described above, the lubricant deterioration sensor 300 enables absorption of the shocks transferred to the substrate attaching portion 30a of the support member 330 from the housing 320 by the shock absorbing members 381, 382, and 383 which are disposed between the housing 320 and the supporting member 330 when the housing 320 is subjected to shocks from the arm 113, and thus it is possible to suppress a possibility of damaging an electronic component mounted on the circuit board 71 which is attached to the substrate attaching portion 30a due to the shocks. Therefore, the lubricant deterioration sensor 300 enables suppression of a failure occurring when the housing 320 is subjected to shocks from the arm 113.

In addition, the lubricant deterioration sensor 300 enables absorption of the shocks transferred to the substrate attaching portion 30a of the support member 330 from the housing 320 by the shock absorbing members 381, 382, and 383 which are disposed between the housing 320 and the supporting member 330 when the housing 320 is subjected to shocks from the arm 113, and thus it is possible to suppress a possibility of changing a positional relationship of the white LED 72 and RGB sensor 73 which are mounted on the circuit board 71 attached on the substrate attaching portion 30a by the shocks. Accordingly, when the housing 320 is subjected to shocks from the arm 113, the lubricant deterioration sensor 300 enables suppression of degrading the detection accuracy of the deterioration of the lubricant 131a of the arm 113.

In addition, since each of the shock absorbing members 381, 382, and 383 includes each of the fixing member adhesion portions 381a, 382a, and 383a and the supporting member adhesion portions 381b, 382b, and 383b, the lubricant deterioration sensor 300 enables not only absorption of the shocks transferred to the supporting member 330 from the housing 320 by the shock absorbing members 381, 382, and 383 but also prevention of the leakage of lubricant 131a from between the housing 320 and the supporting member 330 by the shock absorbing members 381, 382, and 383.

Since an effect other than the above described effects among the effects of the lubricant deterioration sensor 300 is the same as the effect of the lubricant deterioration sensor 200 according to the second embodiment, the description is omitted.

The configuration of an industrial robot as a machine according to the fourth embodiment will be explained.

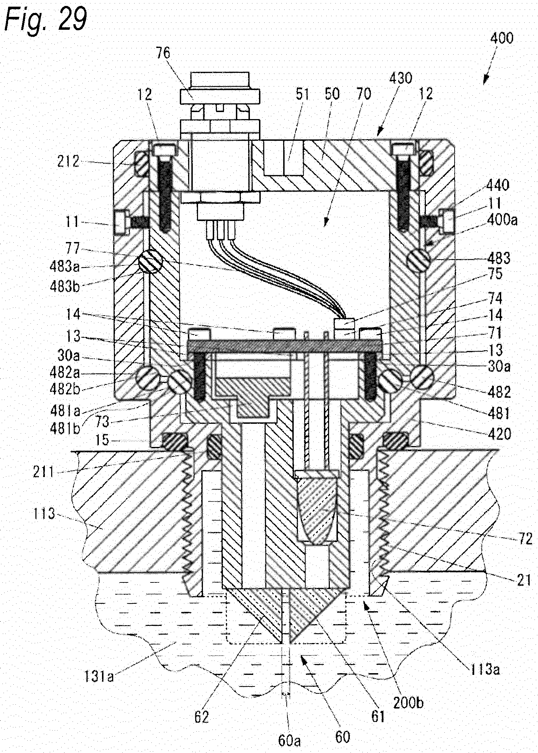

The configuration of the industrial robot according to the embodiment is the same as that of the industrial robot according to the fourth embodiment except that a lubricant deterioration sensor 400 shown in Fig. 29 is provided instead of the lubricant deterioration sensor 300 (refer to Fig. 25). Accordingly, of the components of the industrial robot according to the embodiment, components other than the lubricant deterioration sensor 400 have the same reference numerals as the components of the industrial robot according to the third embodiment and detailed description is omitted.

Fig. 29 is a front sectional view of the lubricant deterioration sensor 400 in a state of being attached to the arm 113.

As shown in Fig. 29, the configuration of the lubricant deterioration sensor 400 is the same as that of the lubricant deterioration sensor 300 (refer to Fig. 25) which includes a housing 420 made of an aluminum alloy for supporting the respective components of the lubricant deterioration sensor 400, a supporting member 430 for supporting a gap forming member 60, and shock absorbing members 481, 482, and 483, which are disposed between the housing 420 and the supporting member 430 and made of soft rubber which absorbs shocks instead of the housing 320 (refer to Fig.

25), the supporting member 330 (refer to Fig. 25) and the shock absorbing members 381, 382, and 383 (refer to Fig. 25).

The supporting member 430 is configured that the supporting member 330 (refer to Fig. 25) includes the holder 440 made of an aluminum alloy instead of a holder 340 (refer to Fig. 25).

The shock absorbing members 481, 482, and 483 are in an annular shape of which the sectional shape is in a circular shape. The shock absorbing members 481, 482, and 483 are disposed in the peripheral space of a substrate attaching portion 400a which is formed by surrounding the substrate attaching portion 30a of the holder 440 between the housing 420 and the holder 440. Each of the shock absorbing members 481, 482, and 483 includes each of the fixing member adhesion portions 481a, 482a, and 483a which is a continuous portion surrounding the supporting member 430 and adheres to the housing 420 and each of the supporting member adhesion portions 481b, 482b, and 483b which is a continuous portion surrounding the supporting member 430 and adheres to the supporting member 430. Meanwhile, the shock absorbing members 481, 482, and 483 may be any material other than the soft rubber as long as it is a material capable of absorbing shocks.

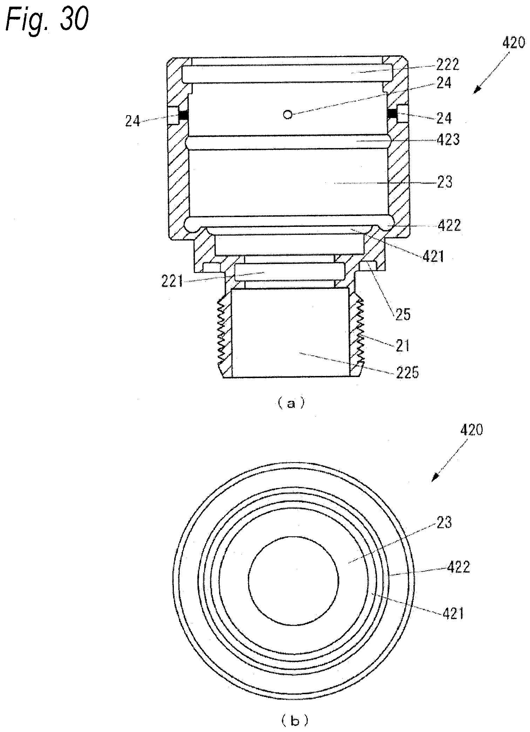

Fig. 30(a) is a front view of the housing 420. Fig. 30(b) is a plan view of the housing 420.

As shown in Fig. 30(a) and Fig. 30(b), the housing 420 is configured to include the annular groove 421 into which the shock absorbing member 481 is fit, the annular groove 422 into which the shock absorbing member 482 is fit, and an annular groove 423 into which the shock absorbing member 483 is fit instead of the groove 321 (refer to Fig. 26(a) and Fig. 26(b)), the groove 322 (refer to Fig. 26(a) and Fig. 26(b)) and the groove 323 (refer to Fig. 26(a) and Fig. 26(b)) in the housing 320 (refer to Fig. 26(a) and Fig. 26(b)).

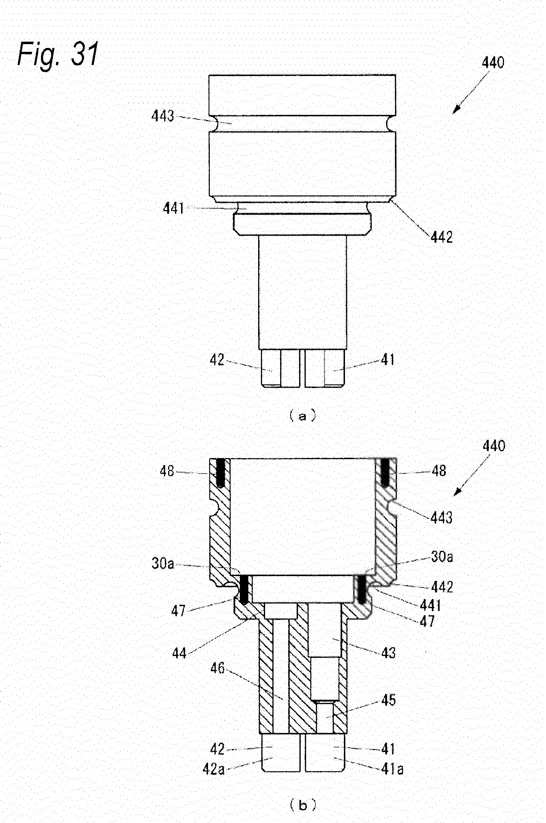

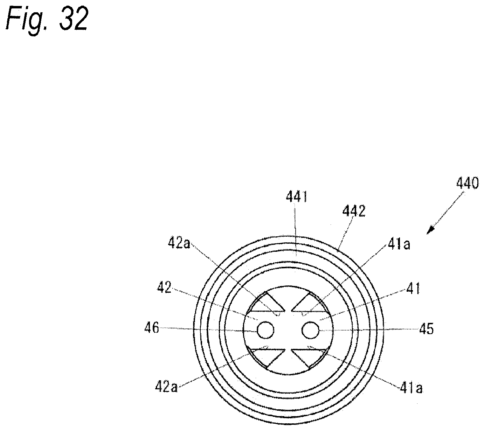

Fig. 31 (a) is a front view of a holder 440. Fig. 31 (b) is a front sectional view of the holder 440. Fig. 32 is a bottom view of the holder 440.

As shown in Fig. 31 (a) to Fig. 32, a holder 440 is configured to include an annular groove 441 into which the shock absorbing member 481 is fit, an annular groove 442 into which the shock absorbing member 482 is fit, and an annular groove 443 into which the shock absorbing member 483 is fit instead of the groove 341 (refer to Fig. 27(a) and Fig. 27(b)), the groove 342 (refer to Fig. 27(a) and Fig. 27(b)) and the groove 343 (refer to Fig. 27(a) and Fig. 27(b)) in the holder 340 (refer to Fig. 27(a) and Fig. 27(b)).

The effect of the lubricant deterioration sensor 400 is the same as the effect of the lubricant deterioration sensor 300 according to the third embodiment, thus the description is omitted.

Further, the mounting position of each of the lubricant deterioration sensors is not limited to that shown in this embodiment, and preferably may be set suitably according to the usage etc. of the industrial robot.

Although the machine according to this invention is the reducer for the industrial robot or the industrial robot in each of the aforesaid embodiments, the machine may be a machine other than the reducer for the industrial robot or the industrial robot.

Reference Signs List

| | |
|---|---|
| 10a | OPTICAL PATH |
| 10b | PERIPHERAL SPACE OF SUBSTRATE ATTACHING PORTION |
| 20 | HOUSING (FIXING MEMBER) |
| 21 | SCREW PORTION (CONTACT PORTION) |
| 30 | SUPPORTING MEMBER |
| 30a | SUBSTRATE ATTACHING PORTION |
| 60 | GAP FORMING MEMBER |
| 60a | OIL THE GAP |
| 71 | CIRCUIT BOARD |
| 72 | WHITE LED (LIGHT EMITTING ELEMENT) |
| 73 | RGB SENSOR (COLOR LIGHT RECEPTION ELEMENT) |
| 80 | SHOCKABSORBING MEMBER |
| 82 | FIXING MEMBER ADHESION PORTION |
| 83 | SUPPORTING MEMBER ADHESION PORTION |
| 100 | INDUSTRIAL ROBOT (MACHINE) |
| 112 to 116 | ARM |
| 113a | SCREW HOLE (HOLE OF MACHINE BODY) |
| 131 | REDUCER (REDUCER FOR INDUSTRIAL ROBOT, MACHINE) |
| 131a | LUBRICANT |
| 132 | REDUCER BODY (MACHINE BODY) |
| 137a, 137b, 139a, 139b | LUBRICANT DETERIORATION SENSOR |

| | |
|---|---|
| 200 | LUBRICANT DETERIORATION SENSOR |
| 200a | PERIPHERAL SPACE OF SUBSTRATE ATTACHING PORTION |
| 220 | HOUSING (FIXING MEMBER) |
| 230 | SUPPORTING MEMBER |
| 281,282 | SHOCK ABSORBING MEMBER |
| 300 | LUBRICANT DETERIORATION SENSOR |
| 300a | PERIPHERAL SPACE OF SUBSTRATE ATTACHING PORTION |
| 320 | HOUSING (FIXING MEMBER) |
| 330 | SUPPORTING MEMBER |
| 381 | SHOCK ABSORBING MEMBER |
| 381a | FIXING MEMBER ADHESION PORTION |
| 381b | SUPPORTING MEMBER ADHESION PORTION |
| 382 | SHOCK ABSORBING MEMBER |
| 382a | FIXING MEMBER ADHESION PORTION |
| 382b | SUPPORTING MEMBER ADHESION PORTION |
| 383 | SHOCK ABSORBING MEMBER |
| 383a | FIXING MEMBER ADHESION PORTION |
| 383b | SUPPORTING MEMBER ADHESION PORTION |
| 400 | LUBRICANT DETERIORATION SENSOR |
| 400a | PERIPHERAL SPACE OF SUBSTRATE ATTACHING PORTION |
| 420 | HOUSING (FIXING MEMBER) |
| 430 | SUPPORTING MEMBER |
| 481 | SHOCKABSORBING MEMBER |
| 481a | FIXING MEMBER ADHESION PORTION |
| 481b | SUPPORTING MEMBER ADHESION PORTION |
| 482 | SHOCKABSORBING MEMBER |
| 482a | FIXING MEMBER ADHESION PORTION |
| 482b | SUPPORTING MEMBER ADHESION PORTION |
| 483 | SHOCKABSORBING MEMBER |
| 483a | FIXING MEMBER ADHESION PORTION |
| 483b | SUPPORTING MEMBER ADHESION PORTION |

## Claims

1. A lubricant deterioration sensor (139b) which is configured to be installed in a machine body (132) and adapted to detect deterioration of lubricant (131a) of the machine body (132), the sensor (139b) comprising:

   a light emitting element (72) configured to emit light;
   a color light reception element (73) configured to detect a color of received light;
   a gap forming member (60) at which an oil gap for allowing the lubricant (131a) to enter therein is formed;
   a supporting member (30;230;330;430) supporting the light emitting element (72), the color light reception element (73), a circuit board (71), and the gap forming member (60);
   a fixing member (20;220;320;420) configured to be fixed to the machine body (132); and
   wherein the gap forming member (60) is configured to transmit the light emitted from the light emitting element (72),
   the oil gap (60a) is disposed on an optical path from the light emitting element (72) to the color light reception element (73),
   the supporting member (30;230;330;430) is provided with a substrate attaching portion (30a) having the circuit board (71) attached thereon,
   **characterized in that**
   the circuit board (71) on which the light emitting element (72) and the color light reception element (73) are mounted;
   a shock absorbing member (80;281,282;381,382,383;481,482,483) disposed between the supporting member (30;230;330;430) and
   the fixing member (20;220;320;420) and configured to absorb shocks; and
   at least a part of the shock absorbing member (80; 281,282;381,382,383;481,482,483) is disposed in a peripheral space of a substrate attaching portion (10b) which surrounds the substrate attaching portion (30a) between the supporting member (30;230;330;430) and the fixing member (20;220;320;420).

2. The lubricant deterioration sensor (139b) according to Claim 1, wherein
the shock absorbing member (80;281,282;381,382,383;481,482,483) includes a fixing member adhesion portion (82;381a,382a,383a;481a,482a,483a) which is a continuous portion surrounding the supporting member (30;230;330;430) and adheres to the fixing member (20;220;320;420) and a supporting member adhesion portion (83;381b,382b,383b;481b,482b,483b), which is an integrated portion formed by surrounding the supporting member (30;230;330;430) and adheres to the supporting member (30;230;330;430).

3. The lubricant deterioration sensor (139b) according to Claim 1, wherein
the fixing member (20;220;320;420) includes a contact portion (21) having an outer side configured to come in contact with the machine body (132),
the supporting member (30;230;330;430) is disposed on an inner side of the contact portion (21) so that at least a part of the optical path (10a) is disposed on the inner side of the contact portion (21), and
at least a part of the shock absorbing member (80; 281,282;381,382,383;481,482,483) is disposed between the contact portion (21) and the supporting member (30;230;330;430).

4. The lubricant deterioration sensor (139b) according to Claim 3, wherein
the contact portion(21) is a portion configured to be inserted into a hole of the machine body (132).

5. The lubricant deterioration sensor(139b) according to Claim 4, wherein
the hole is a screw hole (113a), and
the contact portion (21) is a screw portion (21)which is configured to be inserted into
the screw hole (113a) of the machine body (132) and fixed thereto.

6. A machine (100) comprising:

the lubricant deterioration sensor (139b) and the machine body (132) according to any one of Claims 1 to 5.

7. The machine (100) according to Claim 6, wherein
the machine (100) is a reducer for an industrial robot (131), and
the machine body (132) is a main body of the reducer (131).

8. The machine (100) according to Claim 6, wherein
the machine (100) is the industrial robot,
the macine body (132) is provided with an arm (112-116) and a reducer (131) used at an articular portion of the arm (112-116), and
the lubricant (131a) is lubricant (131a) for the reducer (131).

**Patentansprüche**

1. Sensor (139b) für Verschlechterung von Schmiermittel, der so ausgeführt ist, dass er in einem Maschinen-Körper (132) installiert ist, und so eingerichtet ist, dass er Verschlechterung von Schmiermittel (131a) des Maschinen-Körpers (132) erfasst, wobei der Sensor (139b) umfasst:

ein lichtemittierendes Bauteil (72), das so ausgeführt ist, dass es Licht emittiert;
ein Farblicht-Empfangs-Bauteil (73), das so ausgeführt ist, dass es eine Farbe von empfangenem Licht erfasst;
ein Element (60) zum Ausbilden eines Spalts, an dem ein Öl-Spalt ausgebildet ist, der das Schmiermittel (131a) eintreten lässt;
ein Trageelement (30; 230; 330; 430), das das lichtemittierende Bauteil (72), das Farblicht-Empfangs-Bauteil (73), eine Leiterplatte (71) und das Element (60) zum Ausbilden eines Spalts trägt;
ein Befestigungselement (20; 220; 320; 420), das so ausgeführt ist, dass es an dem Maschinen-Körper (132) befestigt ist; und
wobei das Element (60) zum Ausbilden eines Spalts so ausgeführt ist, dass es das von dem lichtemittierenden Bauteil (72) emittierte Licht durchlässt,
der Öl-Spalt (60a) auf einem optischen Weg von dem lichtemittierenden Bauteil (72) zu dem Farblicht-Empfangs-Bauteil (73) angeordnet ist,

das Trageelement (30; 230; 330; 430) mit einem Träger-Anbringungsabschnitt (30a) versehen ist, an dem die Leiterplatte (71) angebracht ist,

**dadurch gekennzeichnet, dass**

an der Leiterplatte (71) das lichtemittierende Bauteil (72) und das Farblicht-Empfangs-Bauteil (73) montiert sind; ein Stoßdämpf-Element (80; 281, 282; 381, 382, 383; 481, 482, 483) zwischen dem Trageelement (30; 230; 330; 430) und dem Befestigungselement (20; 220; 320; 420) angeordnet ist und so ausgeführt ist, dass es Stöße dämpft; und

wenigstens ein Teil des Stoßdämpf-Elementes (80; 281, 282; 381, 382, 383; 481, 482, 483) in einem Raum am Umfang eines Träger-Anbringungsabschnitts (10b) angeordnet ist, der den Träger-Anbringungsabschnitt (30a) zwischen dem Trageelement (30; 230; 330; 430) und dem Befestigungselement (20; 220; 320; 420) umschließt.

2. Sensor (139b) für Verschlechterung von Schmiermittel nach Anspruch 1, wobei

das Stoßdämpf-Element (80; 281, 282; 381, 382, 383; 481, 482, 483) einen an dem Befestigungselement haftenden Abschnitt (82; 381a, 382a, 383a; 481a, 482a, 483a), der ein durchgehender Abschnitt ist, der das Trageelement (30; 230; 330; 430) umschließt und an dem Befestigungselement (20; 220; 320; 420) haftet, sowie einen an dem Trageelement haftenden Abschnitt (83; 381b, 382b, 383b; 481b, 482b, 483b) enthält, der ein integrierter Abschnitt ist, der mittels Umschließen des Trageelementes (30; 230; 330; 430) ausgebildet wird und an dem Trageelement (30; 230; 330; 430) haftet.

3. Sensor (139b) für Verschlechterung von Schmiermittel nach Anspruch 1, wobei

das Befestigungselement (20; 220; 320; 420) einen Kontaktabschnitt (21) enthält, der eine Außenseite hat, die so ausgeführt ist, dass sie in Kontakt mit dem Maschinen-Körper (132) kommt,

das Trageelement (30; 230; 330; 430) an einer Innenseite des Kontaktabschnitts (21) so angeordnet ist, dass wenigstens ein Teil des optischen Weges (10a) an der Innenseite des Kontaktabschnitts (21) angeordnet ist, und wenigstens ein Teil des Stoßdämpf-Elementes (80; 281, 282; 381, 382, 383; 481, 482, 483) zwischen dem Kontaktabschnitt (21) und dem Trageelement (30; 230; 330; 430) angeordnet ist.

4. Sensor (139b) für Verschlechterung von Schmiermittel nach Anspruch 3, wobei

der Kontaktabschnitt (21) ein Abschnitt ist, der so ausgeführt ist, dass er in ein Loch des Maschinen-Körpers (132) eingeführt wird.

5. Sensor (139b) für Verschlechterung von Schmiermittel nach Anspruch 4, wobei

das Loch ein Schraubenloch (113a) ist, und

der Kontakt-Abschnitt (21) ein Schraubenabschnitt (21) ist, der so ausgeführt ist dass er in das Schraubenloch (113a) des Maschinen-Körpers (132) eingeführt und daran befestigt wird.

6. Maschine (100), die umfasst:

den Sensor (139b) für Verschlechterung von Schmiermittel sowie den Maschinen-Körper (132) nach einem der Ansprüche 1 bis 5.

7. Maschine (100) nach Anspruch 6, wobei

die Maschine (100) ein Untersetzungsgetriebe für einen Industrieroboter (131) ist, und

der Maschinen-Körper (132) ein Hauptkörper des Untersetzungsgetriebes (131) ist.

8. Maschine (100) nach Anspruch 6, wobei

die Maschine (100) der Industrieroboter ist,

der Maschinen-Körper (132) mit einem Arm (112-116) sowie einem Untersetzungsgetriebe (131) versehen ist, das an einem Gelenkabschnitt des Arms (112-116) eingesetzt wird, und

das Schmiermittel (131a) Schmiermittel (131a) für das Untersetzungsgetriebe (131) ist.

**Revendications**

1. Détecteur de détérioration de lubrifiant (139b) configuré pour être installé dans un corps de machine (132) et adapté pour détecter la détérioration du lubrifiant (131a) du corps de machine (132), le capteur (139b) comprenant:

un élément électroluminescent (72) configuré pour émettre de la lumière;

un élément de réception de lumière colorée (73) configuré pour détecter une couleur de lumière reçue;

un élément de formation d'orifice d'entrée (60) au niveau duquel est formé un orifice d'entrée d'huile permettant l'entrée du lubrifiant (131a);

un élément support (30; 230; 330; 430) supportant l'élément électroluminescent (72), l'élément de réception de lumière colorée (73), une carte de circuit (71) et l'élément de formation d'orifice d'entrée (60);

un élément de fixation (20; 220; 320; 420) configuré pour se fixer au corps de machine (132); et

dans lequel l'élément de formation d'orifice d'entrée (60) est configuré pour transmettre la lumière émise par l'élément électroluminescent (72),

l'orifice d'entrée d'huile (60a) est disposé sur un trajet optique entre l'élément électroluminescent (72) et l'élément de réception de lumière colorée (73),

l'élément support (30; 230; 330; 430) est pourvu d'une partie de fixation de substrat (30a) sur laquelle est fixée la carte de circuit imprimé (71),

**caractérisé en ce que**

l'élément électroluminescent (72) et l'élément de réception de lumière colorée (73) sont montés sur la carte de circuit (71);

un élément amortisseur (80; 281 282; 381382383; 481482483) est disposé entre l'élément support (30; 230; 330; 430) et

l'élément de fixation (20; 220; 320; 420) est configuré pour absorber les chocs; et

au moins une partie de l'élément amortisseur (80; 281.282; 381382383; 481482483) est disposé dans un espace périphérique d'une partie de fixation de substrat (10b) qui entoure la partie de fixation de substrat (30a) entre l'élément support (30; 230; 330; 430) et l'élément de fixation (20; 220; 320; 420).

2. Détecteur de détérioration de lubrifiant (139b) selon la revendication 1, dans lequel
l'élément amortisseur (80; 281, 282; 381, 382, 383; 481, 482, 483) comprend une partie d'adhérence d'élément de fixation (82; 381a, 382a, 383a; 481a, 482a, 483A) qui est une partie continue entourant l'élément support (30; 230; 330; 430) et adhère à l'élément de fixation (20; 220; 320; 420) et une partie d'adhérence d'élément support (83; 381b, 382b, 383b; 481b, 482b, 483b) qui est une partie intégrée formée en entourant l'élément support (30; 230; 330 ; 430) et adhère à l'élément support (30; 230; 330; 430).

3. Détecteur de détérioration de lubrifiant (139b) selon la revendication 1, dans lequel
l'élément de fixation (20; 220; 320; 420) comprend une partie de contact (21) avec une face extérieure configurée pour venir en contact avec le corps de machine (132),
l'élément support (30; 230; 330; 430) est disposé sur un côté intérieur de la partie de contact (21) de sorte qu'au moins une partie du trajet optique (10a) est disposée sur le côté intérieur de la partie de contact (21), et
au moins une partie de l'élément amortisseur (80; 281, 282, 381, 382, 383, 481, 482, 483) est disposée entre la partie de contact (21) et l'élément support (30; 230; 330; 430).

4. Détecteur de détérioration de lubrifiant (139b) selon la revendication 3, dans lequel
la partie de contact (21) est une partie configurée pour s'insérer dans un trou du corps de machine (132).

5. Détecteur de détérioration de lubrifiant (139b) selon la revendication 4, dans lequel
le trou est un trou de vis (113a), et
la partie de contact (21) est une partie de vis (21) qui est configurée pour s'insérer dans le trou de vis (113a) du corps de machine (132) et se fixer à celui-ci.

6. Machine (100) comprenant:

le capteur de détérioration de lubrifiant (139b) et le corps de machine (132) selon l'une quelconque des revendications 1 à 5.

7. Machine (100) selon la revendication 6, dans laquelle
la machine (100) est un réducteur pour robot industriel (131), et le corps de machine (132) est un corps principal du réducteur (131).

8. Machine (100) selon la revendication 6, dans laquelle
la machine (100) est le robot industriel,
le corps de machine (132) est pourvu d'un bras (112-116) et d'un réducteur (131) utilisé au niveau d'une partie articulaire du bras (112-116), et
le lubrifiant (131a) est un lubrifiant (131a) pour le réducteur (131).

*Fig. 1*

*Fig. 2*

*Fig. 3*

Fig. 4

# *Fig. 5*

(a)

(b)

*Fig. 6*

( a )

( b )

*Fig. 7*

( a )

( b )

## Fig. 8

(a)

(b)

*Fig. 9*

40

42 — 41

( a )

48 — — 48

30a — 30a

47 — — 47

44 — — 43

46 — — 45

42 — — 41
42a — — 41a

( b )

*Fig. 10*

（a）

（b）

## Fig. 11

(a)

(b)

Fig. 12

# Fig. 13

50

(a)

50

52    51

53          53

(b)

*Fig. 14*

(a)

(b)

*Fig. 15*

(a)

(b)

Fig. 16

COLOR DIFFERENCE ⊿E

DIRECTION OF OPENING OF OIL GAP
WITH RESPECT TO FLOW OF LUBRICANT [° ]

# Fig. 17

(a)

(b)

(c)

## Fig. 18

Fig. 19

( a )

( b )

*Fig. 20*

*Fig. 21*

(a)

(b)

# Fig. 22

( a )

( b )

Fig. 23

(a)

(b)

*Fig. 24*

## Fig. 25

## Fig. 26

(a)

(b)

*Fig. 27*

(a)

(b)

*Fig. 28*

## Fig. 29

## Fig. 30

420

222
24
24
24
423
23
422
421
25
221
21
225

(a)

420

23
422
421

(b)

Fig. 31

( a )

( b )

*Fig. 32*

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 7146233 A
- JP 10104160 A
- US 20100045989 A1
- US 20080024761 A1

**Non-patent literature cited in the description**

- METHOD OF DISCRIMINATING HUE OF CONTAMINANT WITHIN LUBRICANT. **TOMOHIKO YAMAGUCHI.** Faculty of Engineering, Research Report. Fukui University, March 2003, vol. 51, 81-88

- **TOMOMI HONDA.** DETERIORATION DIAGNOSIS OF LUBRICANT·INSPECTION TECHNOLOGY. *Journal of Japan Society for Precision Engineering,* 2009, vol. 75 (3), 359-362